(19)

**Europäisches Patentamt**
**European Patent Office**
**Office européen des brevets**

(11) **EP 4 194 568 A1**

(12) **EUROPEAN PATENT APPLICATION**

(43) Date of publication:
**14.06.2023 Bulletin 2023/24**

(51) International Patent Classification (IPC):
***C12Q 1/70*** *(2006.01)*     ***C12Q 1/6883*** *(2018.01)*

(21) Application number: **21383122.5**

(22) Date of filing: **10.12.2021**

(52) Cooperative Patent Classification (CPC):
**C12Q 1/701; C12Q 1/6883;** C12Q 2600/156

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **GENINCODE UK LIMITED
Manchester M2 3DE (GB)**

(72) Inventors:
• **SORIA FERNÁNDEZ, José Manuel
E-08031 Barcelona (ES)**

• **CARRERA MARTÍNEZ, Marta
E-08173 08173 Sant Cugat del Vallés (Barcelona)
(ES)**
• **WALLS, Matthew
Manchester, M2 3DE (GB)**

(74) Representative: **Marks & Clerk LLP
15 Fetter Lane
London EC4A 1BW (GB)**

Remarks:
The complete document including Reference
Table(s) and the Sequence Listing(s) can be
downloaded from the EPO website

(54) **COVID-19-ASSOCIATED VENOUS THROMBOEMBOLISM**

(57)     A method for the thromboembolic event or disease risk assessment in a subject suffering from COVID-19. The method comprises the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548)), factor XIII Val34Leu (rs5985), factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or ABO blood group rs8176743, and ABO blood group rs8176750, which is indicative of a risk of having or developing a thromboembolic event or disease.

**EP 4 194 568 A1**

**Description**

Technical field

The present invention relates to the field of thromboembolic diseases or disorders. More specifically, it relates to markers and methods for determining whether a subject, particularly a human subject suffering with COVID-19, is at risk of having or developing a thromboembolic disease, disorder, event, or complication, or severe COVID-19.

Background

COVID-19 is one of the most severe public health challenges ever faced. Venous thromboembolic events (VTEs) have emerged as a common complication, increasing disease severity and mortality[1]. Meta-analyses and systematic reviews[1-9] have estimated the incidence of VTE to be 14-31% among patients hospitalized for COVID-19 (7.8-19% for pulmonary embolism and 12-27% for deep venous thrombosis). The true overall incidence is, however, probably higher since some VTEs are asymptomatic and diagnostic tests for VTEs are not routinely requested in clinical practice[11-16]. Certainly, the incidence of VTE is higher in critically ill patients admitted to intensive care units (ICUs).[1,3,5,10]

In the first wave of the pandemic, different medical organizations recommended initiating prophylactic anticoagulation treatment for patients with no contraindications[17-19]. However, such treatment implies a bleeding risk that needs to be taken into account[8,20-26]. Patell et al.[22] reported an incidence of 20.8% for major bleeding in patients with COVID-19, of whom ~40% were receiving anticoagulation therapy. In this regard, around 75 clinical trials are being conducted with the aim of optimizing VTE prophylaxis in patients with COVID-19[28]. These clinical trials, however, face a major limitation: the lack of tools for assessing the risk of VTE. The main clinical biomarker of VTE is D-Dimer, which shows high sensitivity and low specificity.[29] However, when assessing the risk of VTE before haemostatic equilibrium is affected, a single biomarker is of limited use. Many variables affect the appearance of a VTE,[30] both clinical (e.g., obesity, diabetes) and genetic (e.g., Factor V Leiden, prothrombin G20210A).[31,32]

There is, therefore, an urgent need for improved markers and methods for predicting the risk of suffering venous thromboembolic events (VTEs) in patients with COVID-19, including those provided thromboprophylaxis at the moment of hospital admission (as recommended by international organisations).

Summary of the invention

According to an aspect of the present invention, there is provided a method for the thromboembolic event or disease risk assessment in a subject suffering from COVID-19, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548)), factor XIII Val34Leu (rs5985), factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or ABO blood group rs8176743, and ABO blood group rs8176750, which is indicative of a risk of having or developing a thromboembolic event or disease.

According to a further aspect, there is provided a method for predicting the severity of disease in a subject suffering from COVID-19, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or ABO blood group rs8176743, and ABO blood group rs8176750, which is indicative of a risk of developing severe disease.

In an embodiment, the severe disease comprises admission of the subject to an intensive care unit and/or mortality.

In an embodiment, the subject is previously classified as being vulnerable to COVID-19.

According to a further aspect, there is provided a method for identifying a subject suffering from COVID-19 in need of anticoagulant and/or antithrombotic therapy, prophylactic antithrombotic and/or anticoagulant therapy, or a more aggressive program thereof, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), Factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or rs8176743, and ABO blood group rs8176750, which is indicative of being in need of anticoagulant and/or antithrombotic therapy, prophylactic antithrombotic and/or anticoagulant therapy, or a more aggressive program thereof.

In an embodiment, the method further comprises determining one or more risk factor(s) selected from the group consisting of age, sex, body mass index, smoking status, and diabetes mellitus, optionally wherein each of age, sex, body mass index, smoking status, and diabetes mellitus are determined.

In an embodiment, the sample is an oral tissue sample, scraping, or wash or a biological fluid sample, preferably saliva, urine or blood.

In an embodiment, the presence or absence of the polymorphism(s) is identified by allele-specific PCR.

According to a further aspect, there is provided a method of determining the probability of an individual suffering from COVID-19 of presenting a thromboembolic disease or event based on 1 to n variables including the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), Factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176743 or rs8176749, and ABO blood group rs8176750 using the formula:

Probability (Y=1|$x_{1,...,}$ $x_n$) = 1/1 + exp ($\beta_0$ + $\beta_1$ $x_1$ + ... + $\beta_n$ $x_n$), wherein:

- Probability (Y = 1 | $_{X1}$, ..., $X_n$) = probability of presenting a thromboembolic disease or event in a particular individual with concrete and measurable characteristics in a number of variables 1, ...., n, wherein said probability could range between 0 and 1;

- Exp = exponential natural base;

- $\beta_0$ = coefficient that defines the risk (the probability) of thrombosis non related with the variables 1 to n, wherein said coefficient can take a value from - ∞ to + ∞ and is calculated as the natural logarithm of the incidence of venous thrombosis in the population;

- $\beta_1$ = regression coefficient that expresses the risk (higher or lower) to present thrombosis associated with the value/presence of the predictor variable $X_1$, wherein said coefficient can take a value from - ∞ to + ∞;

- $X_1$ = value taken by the predictor variable $X_1$ in an individual, wherein the range of possible values depends on the variable;

- $\beta_n$ = regression coefficient that expresses the risk (higher or lower) to present thrombosis associated with the value/presence of the predictor variable $X_n$, wherein said coefficient can take a value from - ∞ to + ∞;

- $x_n$ = value taken by the predictor variable $x_n$ in an individual, wherein the range of possible values depends on the variable.

In an embodiment, the thromboembolic event or disease is selected from the group of deep vein thrombosis, pulmonary embolism or a combination thereof.

In an embodiment, the subject is admitted to hospital with COVID-19, and optionally administered prophylactic anticoagulation therapy, optionally wherein the prophylactic anticoagulation therapy is administered on admission to hospital.

In an embodiment, a strong linkage disequilibrium is one with an $r^2$ value of more than 0.7, more than 0.8, or more than 0.9, wherein $r^2$ is defined as follows:

$$r^2(p_a, p_b, p_{ab}) = \frac{(p_{ab} - p_a p_b)^2}{p_a(1 - p_a)p_b(1 - p_b)},$$

where $p_{ab}$ is the frequency of haplotypes having allele $\alpha$ at locus 1 and allele $b$ at locus 2

According to a further aspect, there is provided a computer program or a computer-readable media containing means for carrying out any of the methods described.

According to a further aspect, there is provided a kit comprising reagents suitable for detecting in a sample obtained from a subject, the presence or absence of the polymorphisms described herein.

In an embodiment, the reagents comprise primer pairs specific for the amplification of nucleic acid sequences comprising the polymorphisms described herein.

In an embodiment, the kit further comprises reagents suitable for detecting the presence of the SARS-CoV-2 virus.

In an embodiment, the reagents suitable for detecting the presence of the SARS-CoV-2 virus comprise primer pairs specific for the amplification of one or more SARS-CoV-2 nucleic acid sequences.

According to a further aspect, there is provided a method comprising the steps of: providing a kit comprising reagents suitable for detecting, in a sample obtained from a subject, the presence or absence of the polymorphisms described herein; obtaining a results list, said results list indicating the detected presence or absence of said polymorphisms in the sample, detected using said kit; and providing risk information for said subject based upon the detected presence or absence of said polymorphisms.

In an embodiment, the risk information comprises one or more of: a risk of the subject having or developing a thromboembolic event or disease; a risk of the subject developing severe disease; and / or a need of the subject for anticoagulant and/or antithrombotic therapy, prophylactic antithrombotic and/or anticoagulant therapy, or a more aggressive program thereof.

In an embodiment, the risk information is provided to the subject. In another embodiment, the risk information is provided to a clinician.

Brief description of the drawings

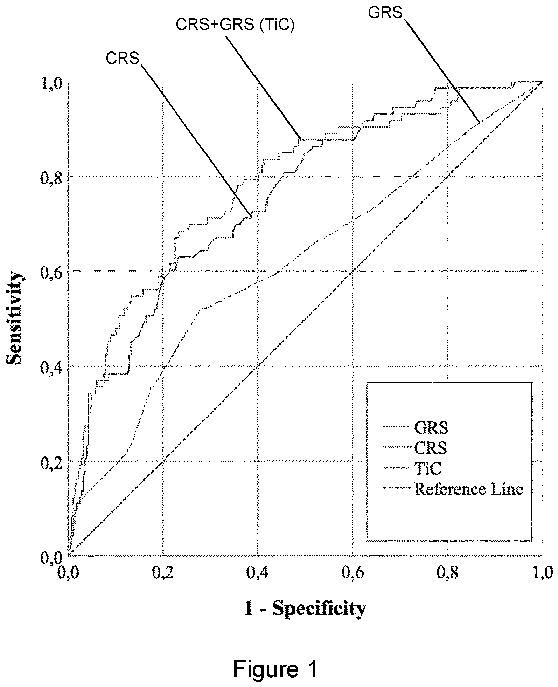

Figure 1 is a graph showing the capacity of different models to predict venous thromboembolic events (VTEs) in subjects selected from the PRECIS_COVID19 cohort. Receiver operating characteristic curves (ROC curves) are shown for each model: clinical variables only (CRS); genetic variables only (GRC); and a combined score based on both the clinical and genetic variables.

Detailed description

We demonstrate herein a risk score based upon the Thrombo inCode (TiC) Score[33]. The score is based on a combination of genetic variants and clinical variables, and is an excellent predictor of the risk of a COVID-19 patient suffering a venous thromboembolic event (VTE). The genetic risk score (GRS) and clinical risk score (CRS) components also have predictive capacity independently. In contrast, the classic genetic thrombophilia model based on the Factor V Leiden (rs6025; chr1:169519049:T:C, F5) and prothrombin G20210A (rs1799963; chr11:46761055:G:A, *F2*) mutations showed no predictive capacity at all. Therefore, according to aspects of the present disclosure, there are provided improved methods of predicting VTE risk in COVID-19 patients, based on said genetic and clinical variables.

A significant association is also demonstrated between suffering a VTE and both mortality ($p<0.001$) and admission to an intensive care unit ($p<0.001$) at 30 and 90 days after hospital admission, highlighting the impact of VTE on the progression and severity of COVID-19. Therefore, according to further aspects of the present disclosure, there are provided methods of predicting severity and/or mortality in COVID-19 patients, based on said genetic and clinical variables.

The present methods allow for the provision of personalized treatment plans, to reduce the incidence of VTE in patients with COVID-19. Treatments may include for example anticoagulant and/or antithrombotic therapy, prophylactic antithrombotic and/or anticoagulant therapy, or a more aggressive program thereof. Anticoagulant therapy may comprise administering anticoagulants such as Low Molecular Weight Heparin (LMWH) or fondaparinux, in case of heparin allergy or heparin-induced thrombocytopenia. LMWH can be administered in a standard prophylactic-, intermediateor therapeutic-intensity dose strategy. When prediction models are used, as for instance, for making treatment decisions, predictive risks may be categorized by using risk cutoff thresholds.

The present methods may also help to inform a more targeted approach to VTE prophylaxis, reducing unnecessary treatments and the associated risks (e.g. bleeding).

The present methods may be used in human subjects with confirmed (e.g. PCR-confirmed) COVID-19. For example those admitted to hospital with COVID-19, and receiving thromboprophylaxis at admission.

*Genetic Variants*

The genetic markers described herein (shown in Table 1 below) have been selected and evaluated after a complex and genuine analysis of thousands of possible markers. To calculate the genetic risk, the cumulative number of risk alleles from those SNPs listed in Table 1 that are present in each individual is considered. For each variant, every individual can have 0, 1 or 2 alleles of risk. On having calculated the sum of risk alleles from the set of the selected variants (n=12) for each individual, a score that could go from 0 to 24 is given. Algorithms for thromboembolic risk estimation are also described herein.

*Table 1. Polymorphisms used by the TiC Score and the present methods*

| Gene | SNP (draft name) | Reference ID of the variant | rs | Informative allele | Other allele | Strand |
|---|---|---|---|---|---|---|
| FXII | 46C>T | FXII, 46C>T | 1801020 | T | C | + |
| ABO blood group (A1 allele) | 261 delG | ABO blood group | 8176719 | G | delG | - |
| | 526C>G | ABO blood group | 7853989 | C | G | + |
| | 703G>A | ABO blood group | 8176743* | G | A | - |
| | (in LD with 703G>A) | ABO blood group | 8176749* | C | T | + |
| | 1059 delC | ABO blood group | 8176750 | C | delC | + |
| SERPIN A10 | 728C>T | Serpin A10, Arg67Stop | 2232698 | T | C | + |
| SERPINC1 | SerpinC1, Ala384Ser (Cambridge II) | SerpinC1, Ala384Ser (Cambridge II) | 121909548 | A | C | - |
| coagulation factor FV | FV Leiden (1746G>A) | FV, R506Q (F5 Leiden) | 6025 | T | C | - |
| | FV Cambridge (1146G>C) | FV, R306T (F5 Cambridge) | 118203906 | C | G | + |
| | FV Hong Kong (1145A>G) | FV, R306G (F5 Hong Kong) | 118203905 | G | A | + |
| coagulation factor XIII (A1 polypeptide) | V34L (226G>T) | FXIII, Val34Leu, | 5985 | C | A | - |
| coagulation factor II | G20210A | Prothrombin, G20210A | 1799963 | A | G | + |
| (*) Only one of those two polymorphisms is used to calculate the A1 allele at the ABO locus. | | | | | | |

In some examples, the genetic risk score is based on the determined presence or absence each of the variants given in Table 1. In other examples, the genetic risk score is based on any combination of one or more of the variants given in Table 1. Some examples of specific combinations of variants which may be used according to aspects of the present disclosure are provided in Table 2 below.

Table 2.

| Combination | Variants analysed |
|---|---|
| 1 | Serpin C1 (antithrombin) Ala384Ser (Cambridge II) (rs121909548)); Factor XIII Val34Leu (rs5985); Factor II (prothrombin) G20210A (rs1799963). |
| 2 | Serpin C1 (antithrombin) Ala384Ser (Cambridge II) (rs121909548)); |
| | Factor XIII Val34Leu (rs5985); Factor II (prothrombin) G20210A (rs1799963); ABO blood group rs8176719; ABO blood group rs7853989; ABO blood group rs8176749 or ABO blood group rs8176743; ABO blood group rs8176750; Factor V Leiden Arg506Gln (rs6025); Serpin A10 (protein Z inhibitor) Arg67Stop (rs2232698). |

(continued)

| Combination | Variants analysed |
|---|---|
| 3 | Serpin C1 (antithrombin) Ala384Ser (Cambridge II) (rs121909548)); Factor XIII Val34Leu (rs5985); Factor II (prothrombin) G20210A (rs1799963); ABO blood group rs8176719; ABO blood group rs7853989; ABO blood group rs8176749 or ABO blood group rs8176743; ABO blood group rs8176750; Factor V Leiden Arg506Gln (rs6025); Serpin A10 (protein Z inhibitor) Arg67Stop (rs2232698); Factor XII C46T (rs1801020). |
| 4 | Serpin C1 (antithrombin) Ala384Ser (Cambridge II) (rs121909548)); Factor XIII Val34Leu (rs5985); Factor II (prothrombin) G20210A (rs1799963); ABO blood group rs8176719; ABO blood group rs7853989; ABO blood group rs8176749 or ABO blood group rs8176743; ABO blood group rs8176750; Factor V Leiden Arg506Gln (rs6025); Serpin A10 (protein Z inhibitor) Arg67Stop (rs2232698); Factor XII C46T (rs1801020); Factor V Cambridge Arg306Thr (rs118203906); Factor V Hong Kong Arg306Gly (rs118203905). |

The detection of one or more SNPs in strong linkage disequilibrium (LD) with any or all of the recited polymorphisms can also be used in place of or in addition to detecting the specifically recited polymorphism. SNPs in LD can be substituted without affecting the magnitude of the association between a GRS and the presence of thromboembolism. In population genetics, linkage disequilibrium (LD) is the non-random association of alleles at different loci in a given population. Loci are said to be in LD when the frequency of association of their different alleles is higher that would be expected if the loci were independent and associated randomly. Measures of LD include the correlation coefficient ($r^2$) and the coefficient of LD (D).

Herein, a strong linkage disequilibrium may be defined by the $r^2$ value. The $r^2$ value is considered particularly suitable to describe linkage disequilibrium.

The $r^2$ measure of linkage disequilibrium is defined as

$$r^2(p_a, p_b, p_{ab}) = \frac{(p_{ab} - p_a p_b)^2}{p_a(1 - p_a)p_b(1 - p_b)},$$

where $p_{ab}$ is the frequency of haplotypes having allele a at locus 1 and allele b at locus 2 (Hill & Robertson. 1968). As the square of a correlation coefficient, $r^2(P_a, P_b, P_{ab})$ can range from 0 to 1 as $p_a$. $p_b$ and $p_{ab}$ vary.

("Hill & Robertson, 1968" is Theor Appl Genetics 1968;38:226-231).

A strong linkage disequilibrium is one with an $r^2$ value of more than 0.7, preferably more than 0.8, more preferably more than 0.9, including e.g. $r^2$ values of 1.

$r^2$ and D values do, however, depend on the frequency of alleles they refer to. Thus, when comparing SNPs with very different allele frequency, both $r^2$ and D values might be low and that does not exclude LD. An alternative measure to take into account the allele frequency (the minor allele frequency or MAF) of the SNPs to be compared is the normalized D or D'.

As an example, SNPs rs8176743 and rs8176749 in the ABO gene are in complete linkage disequilibrium (LD), as both $r^2$ and D' values are '1' or very close to in all studied populations from whom there is available information. The lowest $r^2$ value is 0.937620 and the lowest D' value is 0.999999.

*Detection of Genetic Variants*

Those skilled in the art will readily recognize that the analysis of the nucleotides present in an individual's nucleic acid can be done by any method or technique capable of determining nucleotides present in a polymorphic site. As is obvious in the art, the nucleotides present in the polymorphic markers can be determined from either nucleic acid strand or from both strands.

Once a biological sample from a subject has been obtained (e.g., a bodily fluid, such as urine, saliva, blood (including whole blood), plasma, serum, or a tissue sample, such as a buccal tissue sample or a buccal cell) detection of a sequence variation or allelic variant SNP is typically undertaken. Virtually any method known to the skilled artisan can be employed. Perhaps the most direct method is to actually determine the sequence of either genomic DNA or cDNA and compare these sequences to the known alleles of the gene. This can be a fairly expensive and time-consuming process. Nevertheless, this technology is quite common and is well known.

Any of a variety of methods that exist for detecting sequence variations may be used in the present methods. The particular method used is not important in the estimation of VTE risk or treatment selection.

Other possible commercially available methods exist for the high throughput SNP identification not using direct sequencing technologies, for example, IIIumina's Veracode Technology, Taqman® SNP Genotyping Chemistry and KASPar SNP genotyping Chemistry.

A variation on the direct sequence determination method is the Gene Chip(™) method available from Affymetrix. Alternatively, robust and less expensive ways of detecting DNA sequence variation are also commercially available. For example, Perkin Elmer adapted its TAQman Assay(™) to detect sequence variation. Orchid BioSciences has a method called SNP-IT (™) (SNP-Identification Technology) that uses primer extension with labeled nucleotide analogs to determine which nucleotide occurs at the position immediately 3' of an oligonucleotide probe, the extended base is then identified using direct fluorescence, an indirect colorimetric assay, mass spectrometry, or fluorescence polarization. Sequenom uses a hybridization capture technology plus MALDI-TOF (Matrix Assisted Laser Desorption/Ionization--Time-of-Flight mass spectrometry) to detect SNP genotypes with their MassARRAY(™) system. Promega provides the READIT(™) SNP/Genotyping System (U.S. Pat. No. 6,159,693). In this method, DNA or RNA probes are hybridized to target nucleic acid sequences. Probes that are complementary to the target sequence at each base are depolymerized with a proprietary mixture of enzymes, while probes which differ from the target at the interrogation position remain intact. The method uses pyrophosphorylation chemistry in combination with luciferase detection to provide a highly sensitive and adaptable SNP scoring system. Third Wave Technologies has the Invader OS(™) method that uses proprietary Cleavaseg enzymes, which recognize and cut only the specific structure formed during the Invader process. Invader OS relies on linear amplification of the signal generated by the Invader process, rather than on exponential amplification of the target. The Invader OS assay does not utilize PCR in any part of the assay. In addition, there are a number of forensic DNA testing labs and many research labs that use gene-specific PCR, followed by restriction endonuclease digestion and gel electrophoresis (or other size separation technology) to detect restriction fragment length polymorphisms (RFLPs).

In various examples, the presence or absence of the SNPs is identified by amplifying or failing to amplify an amplification product from the sample. Polynucleotide amplifications are typically template-dependent. Such amplifications generally rely on the existence of a template strand to make additional copies of the template. Primers are short nucleic acids that are capable of priming the synthesis of a nascent nucleic acid in a template-dependent process, which hybridize to the template strand. Typically, primers are from ten to thirty base pairs in length, but longer sequences can be employed. Primers may be provided in double-stranded and/or single-stranded form, although the single-stranded form generally is preferred. Often, pairs of primers are designed to selectively hybridize to distinct regions of a template nucleic acid, and are contacted with the template DNA under conditions that permit selective hybridization. Depending upon the desired application, high stringency hybridization conditions may be selected that will only allow hybridization to sequences that are completely complementary to the primers. In other examples, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

*Polymerase Chain Reaction*

A number of template dependent processes are available to amplify the oligonucleotide sequences present in a given template sample. One of the best known amplification methods is the polymerase chain reaction. In PCR, pairs of primers that selectively hybridize to nucleic acids are used under conditions that permit selective hybridization. The term "primer", as used herein, encompasses any nucleic acid that is capable of priming the synthesis of a nascent nucleic acid in a template-dependent process. Primers may be provided in double-stranded or single-stranded form, although the single-stranded form is preferred. Primers are used in any one of a number of template dependent processes to amplify the target gene sequences present in a given template sample. One of the best known amplification methods is PCR, which is described in detail in U.S. Pat. Nos. 4,683,195, 4,683,202 and 4,800,159, each incorporated herein by reference. In PCR, two primer sequences are prepared which are complementary to regions on opposite complementary strands of the target-gene(s) sequence. The primers will hybridize to form a nucleic-acid:primer complex if the target-gene(s) sequence is present in a sample. An excess of deoxyribonucleoside triphosphates is added to a reaction mixture along with a DNA polymerase, e.g. Taq polymerase that facilitates template-dependent nucleic acid synthesis. If the target-gene(s) sequence:primer complex has been formed, the polymerase will cause the primers to be extended along the target-gene(s) sequence by adding on nucleotides. By raising and lowering the temperature of the reaction mixture, the extended primers will dissociate from the targetgene(s) to form reaction products, excess primers will bind to the target-gene(s) and to the reaction products and the process is repeated. These multiple rounds of amplification, referred to as "cycles", are conducted until a sufficient amount of amplification product is produced.

The amplification product may be digested with a restriction enzyme before analysis. In still other examples, the presence or absence of the SNP is identified by hybridizing the nucleic acid sample with a primer labeled with a detectable moiety. In other examples, the detectable moiety is detected in an enzymatic assay, radioassay, immunoassay, or by detecting fluorescence. In other examples, the primer is labeled with a detectable dye (e.g., SYBR Green I, YO-PRO-I, thiazole orange, Hex, pico green, edans, fluorescein, FAM, or TET). In other examples, the primers are located on a chip. In other examples, the primers for amplification are specific for said SNPs.

In an example, the presence or absence of the genetic variants is identified by allele-specific PCR. This allows for discrimination between the two alleles of a genetic variant. Suitable methods for allele-specific PCR are known to the person skilled in the art. For example, a genotyping assay may comprise PCR primer pairs and probes for allelic discrimination (e.g. with fluorescent reporter dyes). At the end of the PCR reaction, the fluorescent signal for the reporter dyes is measured. The ratio of the signals will be indicative for the genotype of the sample. Alternatively, a common reverse primer may be used with allele-specific forward primers with different tails, and the amplified product used to determine which allele(s) are present in the sample.

Another method for amplification is the ligase chain reaction ("LCR"). LCR differs from PCR because it amplifies the probe molecule rather than producing an amplicon through polymerization of nucleotides. In LCR, two complementary probe pairs are prepared, and in the presence of a target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750, incorporated herein by reference, describes a method similar to LCR for binding probe pairs to a target sequence.

*Isothermal Amplification*

An isothermal amplification method, in which restriction endonucleases and ligases are used to achieve the amplification of target molecules that contain nucleotide 5'-[[alpha]-thio]-triphosphates in one strand of a restriction site also may be useful in the amplification of nucleic acids in the present methods. In one example, loop-mediated isothermal amplification (LAMP) method is used for single nucleotide polymorphism (SNP) typing.

*Strand Displacement Amplification*

Strand Displacement Amplification (SDA) is another method of carrying out isothermal amplification of nucleic acids which involves multiple rounds of strand displacement and synthesis, i.e., nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection.

*Transcription-Based Amplification*

Other nucleic acid amplification procedures include transcription-based amplification systems, including nucleic acid sequence based amplification. In nucleic acid sequence based amplification, the nucleic acids are prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer, which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by a polymerase such as T7 or SP6. In an isothermal cyclic reaction, the RNA's are reverse transcribed into double stranded DNA, and transcribed once against with a polymerase such as T7 or SP6. The resulting products, whether truncated or complete, indicate target specific sequences.

Other amplification methods may be used in accordance with the present methods. In one examples, "modified" primers are used in a PCR-like, template and enzyme dependent synthesis. The primers may be modified by labelling with a capture moiety (e.g., biotin) and/or a detector moiety (e.g., enzyme). In the presence of a target sequence, the probe binds and is cleaved catalytically. After cleavage, the target sequence is released intact to be bound by excess probe. Cleavage of the labelled probe signals the presence of the target sequence. In another approach, a nucleic acid amplification process involves cyclically synthesizing single-stranded RNA ("ssRNA"), ssDNA, and double-stranded DNA (dsDNA), which may be used in accordance with the present methods. The ssRNA is a first template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a second template for a second primer, which also includes the sequences of an RNA polymerase promoter (exemplified by T7 RNA polymerase) 5' to its homology to the template. This primer is then extended by DNA polymerase (exemplified by the large "Klenow" fragment of *E. coli* DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

*Methods for Nucleic Acid Separation*

It may be desirable to separate nucleic acid products from other materials, such as template and excess primer. In one example, amplification products are separated by agarose, agarose-acrylamide or polyacrylamide gel electrophoresis using standard methods (Sambrook et al., 1989, see infra). Separated amplification products may be cut out and eluted from the gel for further manipulation. Using low melting point agarose gels, the separated band may be removed by heating the gel, followed by extraction of the nucleic acid. Separation of nucleic acids may also be effected by chromatographic techniques known in the art. There are many kinds of chromatography which may be used in the practice of the present methods, including adsorption, partition, ionexchange, hydroxylapatite, molecular sieve, reverse-phase, column, paper, thin-layer, and gas chromatography as well as HPLC. In certain examples, the amplification products are visualized. A typical visualization method involves staining of a gel with ethidium bromide and visualization of bands under UV light. Alternatively, if the amplification products are integrally labeled with radio- or fluorometrically-labeled nucleotides, the separated amplification products can be exposed to X-ray film or visualized with light exhibiting the appropriate excitatory spectra.

Alternatively, the presence of the polymorphic positions according to the present methods can be determined by hybridisation or lack of hybridisation with a suitable nucleic acid probe specific for a polymorphic nucleic acid but not with the non-mutated nucleic acid. By "hybridize" is meant a pair to form a double-stranded molecule between complementary polynucleotide sequences, or portions thereof, under various conditions of stringency. For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred example, hybridization will occur at 30°C in 750 mM NaCl, 75 mM trisodium citrate, and 1% SDS. In a more preferred example, hybridization will occur at 37°C in 500 mM NaCl, 50 mM trisodium citrate, 1% SDS, 35% formamide, and 100 [mu]g/ml denatured salmon sperm DNA (ssDNA). In a most preferred example, hybridization will occur at 42°C in 250 mM NaCl, 25 mM trisodium citrate, 1% SDS, 50% formamide, and 200 [mu]g/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred example, wash steps will occur at 25°C in 30 mM NaCl, 3 mM trisodium citrate, and 0.1% SDS. In a more preferred example, wash steps will occur at 42°C in 15 mM NaCl, 1.5 mM trisodium citrate, and 0.1% SDS. In a more preferred example, wash steps will occur at 68°C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196: 180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York, 1989.

Nucleic acid molecules useful for hybridisation in the present methods include any nucleic acid molecule which exhibits substantial identity so as to be able to specifically hybridise with the target nucleic acids. Polynucleotides having "substantial identity" to an endogenous sequence are typically capable of hybridizing with at least one strand of a double-stranded nucleic acid molecule. By "substantially identical" is meant a polypeptide or nucleic acid molecule exhibiting at least 50% identity to a reference amino acid sequence or nucleic acid sequence. Preferably, such a sequence is at least 60%, more preferably 80% or 85%, and more preferably 90%, 95% or even 99% identical at the amino acid level or nucleic acid to the sequence used for comparison. Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e<"3> and e<"100> indicating a closely related sequence.

A detection system may be used to measure the absence, presence, and amount of hybridization for all of the distinct sequences simultaneously. Preferably, a scanner is used to determine the levels and patterns of fluorescence.

Another method for detecting sequence variations is based on the amplification by PCR of specific human targets and the subsequent detection of their genotype by hybridization to specific HairLoop™ probes spotted on a microarray. HairLoop™ is a stem-loop, single-stranded DNA molecule consisting of a probe sequence embedded between complementary sequences that form a hairpin stem. The stem is attached to the microarray surface by only one of its strands. In the absence of a DNA target, the HairLoop™ is held in the closed state. When the target binds perfectly (no mismatch) to its HairLoop™, the greater stability of the probe-target duplex forces the stem to unwind, resulting in an opening of the HairLoop™. Due to these unique structural and thermodynamic properties, HairLoop™ offer several advantages over linear probes, one of which is their increased specificity differentiating between two DNA target sequences that differ by as little as a single nucleotide. HairLoop™ act like switches that are normally closed, or "off". Binding to fluorescent DNA target induces conformational changes that open the structure and as a result after washing, the fluorescence is visible, or "on". One HairLoop™ is designed to be specific to one given allele. Thus, assessment of a point mutation for a bi-allelic marker requires two HairLoop™; one for the wild-type allele, and one for the mutant allele.

Flanking sequences which may be used for the detection of the polymorphisms described in table 1 are given in table 3. Where suitable, these sequences may be used by any of the detection methods described above, for example allele-specific PCR or HairLoop™ technology.

In addition to these sequences, the sequence surrounding the polymorphism of rs8176749 is: GAGCACCTTGGTGGGTTTGTGGCGCAGCAGGTACTTGTTCAGGTGGCTCTCGT (SEQ ID NO: 25). Here the bold underlined C residue is the allele of risk, whereas a T in this position has a neutral effect. This is at position 133255801 on chromosome 9 (GRCh38).

Table 3

| SEQ ID NO: | Variant name | Sequence comprising the polymorphism | Allele | dbSNP accession number | Chrom. | Position in chrom | Strand |
|---|---|---|---|---|---|---|---|
| 1 | FXII, 46 C>T | GGACGGA**T**GCCATGA | Risk | rs1801020 | 5 | 176836532 | + |
| 2 | | GGACGGA**C**GCCATGA | Non-risk | | | | |
| 3 | ABO, 261delG | CTCGTGGT**G**ACCCCT | Risk | rs8176719 | 9 | 136132908 | - |
| 4 | | CTCGTGGT**C**ACCCCTT | Non-risk | | | | |
| 5 | ABO, 526C>G | GGAGGTG**C**GCGCCT | Risk | rs7853989 | 9 | 136131592 | + |
| 6 | | GGAGGTG**G**GCGCCT | Non-risk | | | | |
| 7 | ABO, 703G>A | TGCACCCC**G**GCTTCTAC | Risk | rs8176743 | 9 | 136131415 | - |
| 8 | | TGCACCCC**A**GCTTCTAC | Non-risk | | | | |
| 9 | ABO,1059delC | TCCGGAAC**C**CGTGAGC | Risk | rs8176750 | 9 | 136131059 | + |
| 10 | | TCCGGAA_CCGTGAGCG | Non-risk | | | | |
| 11 | SERPINA10, 728 C>T | CCTGCTG**T**GAAAGATCT | Risk | rs2232698 | 14 | 94756669 | + |
| 12 | | CCTGCTG**C**GAAAGATCT | Non-risk | | | | |
| 13 | SERPINC1, Ala384Ser (Cambridge II) | CGGTACTTG**A**AGCTGCTT | Risk | rs121909548 | 1 | 173873176 | - |
| 14 | | CGGTACTTGCAGCTGCTT | Non-risk | | | | |
| 15 | FV, R506Q (FV Leiden) | ATTCCT**T**GCCTGTCC | Risk | rs6025 | 1 | 169519049 | - |
| 16 | | ATTCCT**C**GCCTGTCC | Non-risk | | | | |
| 17 | FV, R306T (FV Cambrigde) | GAAAACCA**C**GAATCTTAAG | Risk | rs118203906 | 1 | 169524537 | + |
| 18 | | GAAAACCA**G**GAATCTTAAG | Non-risk | | | | |
| 19 | FV, R306G (FV Hong Kong) | AAGAAAACC**G**GGAATCTTA | Risk | rs118203905 | 1 | 169524536 | + |
| 20 | | AAGAAAACC**A**GGAATCTTA | Non-risk | | | | |
| 21 | FXIII, Va)34Leu | GGGCACCA**C**GCCCTGA | Risk | rs5985 | 6 | 6318795 | |
| 22 | | GGGCACCA**A**GCCCTGA | Non-risk | | | | |
| 23 | Prothrombin, G20210A | TCTCAGC**A**AGCCTCAAT | Risk | rs1799963 | 11 | 46761055 | + |
| 24 | | **TCTCAGCGAGCCTCAAT** | Non-risk | | | | |

An exemplary algorithm for estimating the risk of developing and/or suffering a thromboembolic event is shown as function 1 below.

*Function 1*

Estimating the risk of thrombosis.
The individual estimation of the risk of thrombosis is based on a logistic regression model. The aim of this model is to calculate the probability that a person has of presenting venous thrombosis according to his/her genetic, sociodemographic and clinical characteristics. To calculate this probability we use the following equation:

$$\text{Probability } (Y=1|x_{1,\dots,} x_n) = 1 \,/\, 1 + \exp (\beta_0 + \beta_1 x_1 + \dots + \beta_n x_n),$$

wherein:

- Probability ($Y = 1 \mid x_1, \dots, x_n$) = probability of presenting a thrombosis in a particular individual with concrete and measurable characteristics in a number of variables 1, ...., n. This probability could range between 0 and 1;
- Exp = exponential natural base;
- $\beta_0$ = coefficient that defines the risk (the probability) of thrombosis non related with the variables 1 to n. This coefficient can take a value from $-\infty$ to $+\infty$ and is calculated as the natural logarithm of the incidence of venous thrombosis in the population;
- $\beta_1$ = regression coefficient that expresses the risk (higher or lower) to present thrombosis associated with the value/presence of the predictor variable $x_1$. This coefficient can take a value from $-\infty$ to $+\infty$;
- $X_1$ = value taken by the predictor variable $X_1$ in an individual. The range of possible values depends on the variable;
- $\beta_n$ = regression coefficient that expresses the risk (higher or lower) to present thrombosis associated with the value/presence of the predictor variable $x_n$. This coefficient can take a value from $-\infty$ to $+\infty$;
- $x_n$ = value taken by the predictor variable $x_n$ in an individual. The range of possible values depends on the variable.

Variables 1 to n comprise variables representing the presence or absence of the polymorphisms and clinical variables described herein. $\beta$ values $\beta_1$ to $\beta_n$ may be selected from those provided in table 4 accordingly.
In some examples, the model may include the effect of the combination of some variables in terms of interaction or modification of the effect. That is, the effect size (Beta value) of a single variable ($x_f$) can be $\beta_f$ but if this variable is present in combination with another variable ($x_g$) the effect size may vary (increase or decrease) and therefore to consider the effect size of the variable $x_f$ we can consider not only the $\beta_f$ but also a second effect size $\beta_{f \cdot g}$ by adding the $\beta_f$ and the $\beta_{f \cdot g}$. Thus:

*Function 2*

$$\text{Probability } (Y=1|x_{1,\dots,} x_n) = 1 \,/\, 1 + \exp (\beta_0 + \beta_1 x_1 + \dots + \beta_n x_n + \beta_{f \cdot g} x_f \cdot x_g + \dots + \beta_{h \cdot i} x_h \cdot x_i),$$

wherein:

- Probability ($Y = 1 \mid x_1, \dots, x_n$) = probability of presenting a thrombosis in a particular individual with concrete and measurable characteristics in a number of variables 1, ...., n. This probability could range between 0 and 1;
- Exp = exponential natural base;
- $\beta_0$ = coefficient that defines the risk (the probability) of thrombosis non related with the variables 1 to n. This coefficient can take a value from $-\infty$ to $+\infty$ and is calculated as the natural logarithm of the incidence of venous thrombosis in the population;
- $\beta_1$ = regression coefficient that expresses the risk (higher or lower) to present thrombosis associated with the value/presence of the predictor variable $x_1$. This coefficient can take a value from $-\infty$ to $+\infty$;
- $X_1$ = value taken by the predictor variable $X_1$ in an individual. The range of possible values depends on the variable;
- $\beta_n$ = regression coefficient that expresses the risk (higher or lower) to present thrombosis associated with the value/presence of the predictor variable $X_n$. This coefficient can take a value from $-\infty$ to $+\infty$;
- $x_n$ = value taken by the predictor variable $x_n$ in an individual. The range of possible values depends on the variable;
  $\beta_{f \cdot g}$ = regression coefficient that expresses the risk (higher or lower) to present thrombosis associated with the combined presence of the predictor variables $x_f$ and $x_g$. This coefficient can take a value from $-\infty$ to $+\infty$;

- $X_f$ = value taken by the predictor variable $x_f$ in an individual. The range of possible values depends on the variable;
- $x_g$ = value taken by the predictor variable $x_g$ in an individual. The range of possible values depends on the variable;
- $P_{h\cdot i}$ = regression coefficient that expresses the risk (higher or lower) to present thrombosis associated with the combined presence of the predictor variables $x_h$ and $x_i$. This coefficient can take a value from $-\infty$ to $+\infty$;
- $x_h$ = value taken by the predictor variable $x_h$ in an individual. The range of possible values depends on the variable;
- $x_i$ = value taken by the predictor variable $x_i$ in an individual. The range of possible values depends on the variable;

Variables which may be included in the model and the beta values and odds ratios of each of these variables are shown in Table 4. Genetic variants rs118203906 (Factor V Cambridge) and rs118203905 (Factor V Hong Kong) may also be included in the model.

Table 4.

| Variables | Beta value | Odds ratio | Odds ratio 95% CI | |
| --- | --- | --- | --- | --- |
| | | | lower | upper |
| **Clinical variables** | | | | |
| age: continuous | 0.039 | 1.039 | 1.017 | 1.062 |
| gender (male) | 0.373 | 1.452 | 0.788 | 2.675 |
| BMI: >30 | 1.527 | 4.604 | 2.018 | 10.505 |
| smoking | -0.233 | 0.792 | 0.339 | 1.851 |
| diabetes type 2 | 1.301 | 3.674 | 1.329 | 10.161 |
| **Genetic variants** | | | | |
| FV Leiden - Heterozygote | 0.252 | 1.287 | 0.246 | 6.732 |
| F2 - Heterozygote | 0.946 | 2.576 | 0.218 | 30.416 |
| ABO A1 | 0.264 | 1.301 | 0.75 | 2.258 |
| Serpin C1 | 2.118 | 8.311 | 1.851 | 37.311 |
| Serpin A10 | 0.027 | 1.028 | 0.103 | 10.257 |
| F12 | -0.207 | 0.813 | 0.471 | 1.403 |
| F13 | 0.576 | 1.778 | 1.043 | 3.032 |

Table 5. Definition of A1 allele

| The subject carries at least one A1 allele at ABO locus if any of the following combinations is present: | | | | | |
| --- | --- | --- | --- | --- | --- |
| | **Genotypes** | | | | |
| **Combination** | **rs8176719** | **rs7853989** | **rs8176743\*** | **rs8176749\*** | **rs8176750** |
| 1 | GG | CC | GG | CC | CC |
| 2 | GG | CC | GG | CC | CdelC |
| 3 | GG | CG | GA | CT | CC |
| 4 | GdelG | CC | GG | CC | CC |
| 5 | GG | CG | GG | CC | CC |
| (*) Only one of those two polymorphisms is used to calculate the A1 allele at ABO locus. | | | | | |

Surprisingly, the combination of SNP markers included in the present methods and set forth in table 1 and using the functions described herein have proved to be capable to establishing the risk to develop a thromboembolic disease or event in a COVID-19 patient with a higher accuracy than that obtained using the methods nowadays in use or published functions including genetic information.

Surprisingly, the combination of SNP markers included in the present methods and set forth in table 1 and using the functions described herein have proved to be capable of predicting the risk of a thromboembolic disease or event in a COVID-19 patient with a higher accuracy than that obtained using the methods nowadays in use or published functions including genetic information.

By the use of the methods described, a personalized risk is obtained for the development of a thromboembolic event in a COVID-19 patient, in particular deep vein thrombosis and/or pulmonary embolism.

*Definitions*

"Thromboembolic event" in the context of this application should be understood as the alteration of the hemostasis that leads to the development of a blood clot (thrombus) inside a vascular vessel (artery or vein). The thrombus can even obstruct the vascular vessel completely and/or become detached and obstruct another vascular vessel.

"Thromboembolic event" may include among others the following conditions: arterial thrombosis, fatal- and non-fatal myocardial infarction, stroke, transient ischemic attacks, cerebral venous thrombosis, peripheral arteriopathy, deep vein thrombosis and pulmonary embolism, in particular deep vein thrombosis and/or pulmonary embolism

"Thromboembolic event" in the context of this application is used interchangeably with "thromboembolism".

"Thromboembolic event" in the context of this application is used interchangeably with "thrombosis".

"Thromboembolic event" in the context of this application is used interchangeably with "thromboembolic complication".

"Thrombophilia" in the context of this application should be understood as the disorders of haemostasis that predispose to thrombosis. Included are heritable deficiencies of the natural anticoagulants antithrombin, protein C, and protein S and common mutations in the genes encoding clotting factors and acquired thrombophilias such as antiphospholipid antibodies.

The terms "disease" and "disorder" shall be interpreted in the context of this application interchangeably.

"Mutation" in the context of this application should be understood as the change of the structure of a gene, resulting in a variant form which may be transmitted to subsequent generations, caused by the alteration of single base units in DNA, or the deletion, insertion, or rearrangement of larger sections of genes or chromosomes.

"Genetic variants" in the context of this application refers to genetic differences both within and among populations. There may be multiple variants of any given gene in the human population, leading to polymorphism.

The terms "polymorphism" and "single nucleotide polymorphism" (SNP) are used herein interchangeably and relate to a nucleotide sequence variation occurring when a single nucleotide in the genome or another shared sequence differs between members of species or between paired chromosomes in an individual. A SNP can also be designated as a mutation with low allele frequency greater than about 1% in a defined population. Single nucleotide polymorphisms according to the present application may fall within coding sequences of genes, non-coding regions of genes or the intronic regions between genes.

The term "sample", as used herein, refers to any sample from a biological source and includes, without limitation, cell cultures or extracts thereof, biopsied material obtained from a mammal or extracts thereof, and blood, saliva, urine, feces, semen, tears, or other body fluids or extracts thereof.

Example

There is an urgent need to be able to predict the risk of suffering venous thromboembolic events (VTEs) in patients with COVID-19, including those provided thromboprophylaxis at the moment of hospital admission (as recommended by international organisations). The present retrospective study examines whether the Thrombo inCode (TiC) score, a validated clinical-genetic score for assessing the risk of VTE in the general population, could provide the necessary tool. The TiC score (which takes into account the genetic variants rs6025, rs118203905, rs118903906, rs1799963, rs121909548, rs1801020, rs5985, rs2232698 and 4 genetic variants providing the ABO::ABOaA1 haplotype, plus gender, age, obesity, smoking habit, and diabetes), and other genetic and clinical scores, were calculated for a cohort of 352 patients with PCR-confirmed COVID-19, all of whom received thromboprophylaxis at admission, and among whom 73 went on to experience a VTE. A significant association was detected between the suffering of a VTE and both mortality (p<0.001) and admission to an intensive care unit (p<0.001), highlighting the impact of VTE on the progression and severity of COVID-19. Comparison of the calculated risk scores indicated that the Factor V Leiden and prothrombin mutations model showed no useful predictive capacity (AUC 0.51, p>0.05), while that of the TiC score was excellent (AUC 0.78; p<0.0001; OR 7.16 [5.6-43.2]). Certainly, the TiC score performed better than its clinical or genetic components alone (AUC 0.75 and AUC 0.61, respectively), and showed that 68.5% of the VTEs that occurred may have been prevented with more intense prophylaxis. The positive predictive value of the TiC Score was 43.5, and its negative predictive value 90.3, with a 'number needed to treat' of 2.3. In summary, the TiC score, which has been validated for use in different populations, is of use in stratifying patients with COVID-19 at risk of a VTE. It could therefore guide clinical decisions regarding the optimal intensity of anticoagulation treatment at hospital admission. Such personalized therapy could have a substantial impact on the morbidity and mortality of patients with COVID-19.

**Patients and Methods**

*The PRECIS_CQVID19 cohort*

This study was performed using data from patients involved in the PRECIS_COVID19 study at the *Hospital de la Santa Creu i Sant Pau* (Barcelona, Spain). All procedures were approved by the Institutional Review Board and adhered to the Declaration of Helsinki.

The PRECIS_COVID19 cohort consists of 734 patients, all aged over 18 years, with a confirmed diagnosis of COVID-19, all of whom were admitted to the above hospital between April and July 2020. COVID-19 was confirmed by real-time reverse-transcription PCR assays using nasal and pharyngeal swabs. All patients were administered standard thromboprophylactic treatment at the time of their admission to hospital following international recommendations[17-19] and our institute's protocol (March 2020).

Since diagnostic tests for VTE were performed only when an event was strongly suspected, D-Dimer levels were used to identify patients in which a VTE could be ruled out. Recent studies on COVID-19 patients[35,36] have shown that if D-Dimer levels are below 1000 ng/mL, a VTE can be discounted (negative predictive value [NPV] 98-100%). A total of 279 patients had a D-Dimer value below this cut-off, and were thus considered as non-VTE (control) patients. Of the remainder, 382 patients had a D-Dimer levels of >1000 ng/mL but showed no medical evidence of having suffered a VTE (these patients were excluded from further analyses), and 73 suffered a VTE event during hospitalization (either a deep venous thrombosis or a pulmonary embolism). Diagnoses were confirmed using Doppler ultrasonography, magnetic resonance, arteriography, phlebography, pulmonary gammagraphy and computed tomography pulmonary angiography. The total number of subjects in the study was therefore n = 352 (279 + 73).

*Blood collection and genotyping*

Blood samples were obtained by venipuncture at admission. DNA was extracted from whole blood by digestion and selective precipitation with ethanol using the QIAamp DNA-blood kit (Qiagen, Düsseldorf, Germany), according to the manufacturer's instructions.

The prothrombotic genetic variants included in the TiC score were genotyped using the Thrombo inCode kit (TiC, GENinCode Plc, Oxford, UK); this was performed using a CFX96™ Real-Time System (Bio-Rad, Hercules, California, USA).

*Risk Scores*

In addition to the TiC score, the predictive capacities of its clinical and genetic components were independently assessed. The capacity of the classical Factor V Leiden + prothrombin G20210A mutations model was also examined, both alone and in combination with clinical risk. A total of five models were therefore compared, the details of which are described below:

*1. TiC model.* A combination of the variables in both the Genetic Risk Score (GRS) and the Clinical Risk Score (CRS) described below. The TiC model therefore takes into account 12 genetic variants (rs6025, rs118203905, rs118203906, rs1799963, rs121909548, rs1801020, rs5985, rs2232698 and 4 genetic variants reporting the ABO:A1 haplotype), plus age, sex, obesity, smoking habit and diabetes. The original TiC model includes other clinical variables (such as family history, oral contraceptive use and pregnancy), but these were not evaluated as they were difficult to obtain in the COVID19 context.

*2. Genetic Risk Score* (GRS). The twelve genetic variants reported by Soria et al.[33], including the variants rs6025, rs118203905, rs118203906, rs1799963, rs121909548 (chr1:173873176:C:A, *SERPINC1),* rs1801020 (chr5:176836532:A:G, *F12),* rs5985 (chr6:6318795:C:A, *F13),* rs2232698 (chr14:94756669:G:A, *SERPINE10*) and 4 variants providing the ABO:A1 haplotype (additive effect of A1 allele).

*3. Clinical Risk Score* (CRS). Five clinical variables were assessed: age, sex, obesity, smoking habit, and diabetes. These variables have been shown to be associated with VTE and have been reported to be useful in estimating VTE risk[37-39]. Smoking habit was codified as a dichotomic variable (smoker/non-smoker); obesity was defined as body mass index >30.

4. FVL+PT score. The classic genetic thrombophilia model based on the Factor V Leiden (rs6025; chr1:169519049:T:C, F5) and prothrombin G20210A (rs1799963; chr11:46761055:G:A, F2) mutations.

5. FVL+PT + CRS. Combination of the variables in the FVL+PT and the CRS model explained before; it therefore includes the variants rs6025 and rs1799963, plus age, sex, obesity, smoking habit and diabetes.

*Statistical Analysis*

A descriptive analysis of both the genetic and clinical variables was performed, and the relationship with VTE assessed by the Chi-squared test for bivariate associations. The same method was used to evaluate the association between ICU admission and mortality rates (at 30 and 90 days after hospital admission), as well as the association between VTE and mortality rates and ICU admission. Significance was set at p<0.05. All risk models were constructed by including the corresponding variables as additive linear predictors of VTE using logistic regression. The predictive capacity of the different models was examined using receiver operating curves (ROC)[40], employing the optimal cut-off based on the Youden index[41]. The significance of the predictive capacity of each score was measured by comparing with a random model (area under the ROC curve [AUC] of 0.5), using the DeLong test[42].

As well as determining test sensitivity and specificity, the negative and positive predictive values (NPV and PPV) of each model were determined, along with the positive and negative likelihood ratios (LR), and the odds ratio (OR).

All calculations were performed using SPSS v.26.0 software (Released 2019) (IBM Corp., Armonk, NY, USA).

**Results**

*Descriptive Analysis*

Table 6 provides the descriptive analysis of the clinical and genetic variables of all 352 patients. The associations between VTE and four clinical variables (age, sex, obesity and smoking habit), and the genetic variants rs121909548 in *SERPINC1* and rs5985 in *F13,* were significant (p<0.05).

*Table 6. Descriptive analysis of the clinical and genetic variables in the PRECIS_COVID19 cohort. Frequency and distribution of clinical variables, and frequencies of both reference and risk alleles for the twelve genetic variants. The P-value (p) refers to the association with venous thromboembolic events (VTE) (bivariate analysis).*

| Clinical and genetic (*) variables | | PRECIS_CQVID19 (n=352) | Patients with VTE (n=73) | Non-VTE Controls (n=279) | P |
|---|---|---|---|---|---|
| Age (years), mean (SD) | | 59.0 (15.0) | 64.9 (11.5) | 57.5 (15.4) | <0.001 |
| Sex (female), n (%) | | 158 (44.9) | 25 (34.2) | 133 (47.7) | 0.040 |
| Obesity, n (%) | | 38 (10.8) | 21 (28.8) | 17 (6.1) | <0.001 |
| Smoking, n (%) | | 24 (6.8) | 15 (20.5) | 9 (3.2) | <0.001 |
| Diabetes, n (%) | | 47 (13.4) | 14 (19.2) | 33 (11.8) | 0.100 |
| ABO:A1, n (%) | 0 | 221 (62.8) | 43 (58.9) | 178 (63.8) | |
| | 1/2 | 131 (37.2) | 30 (41.1) | 101 (36.2) | 0.411 |
| rs6025, n (%) F5 | 0 | 344 (97.7) | 71 (97.3) | 273 (97.8) | |
| | 1 | 8 (2.3) | 2(2.7) | 6(2.2) | 1.000 |
| rs1799963, n (%) F2 | 0 | 349 (99.3) | 72 (98.6) | 277 (99.3) | |
| | 1 | 3 (0.7) | 1(1.4) | 2 (0.7) | 1.000 |
| rs121909548, n (%) SERPINC1 | 0 | 344 (98) | 68 (93.2) | 276 (98.9) | |
| | 1 | 8 (2.0) | 5 (6.8) | 3 (1.1) | 0.011 |
| rs1801020, n (%) F12 | 0 | 204 (58.0) | 46 (63.0) | 158 (56.6) | |
| | 1/2 | 148 (42.0) | 27 (37.0) | 121 (43.4) | 0.325 |

(continued)

| Clinical and genetic (*) variables | | PRECIS_CQVID19 (n=352) | Patients with VTE (n=73) | Non-VTE Controls (n=279) | P |
|---|---|---|---|---|---|
| rs5985, n (%) F13 | 0 | 200 (56.8) | 34 (46.6) | 166 (59.5) | |
| | 1/2 | 152 (43.2) | 39 (53.4) | 113 (40.5) | 0.047 |
| rs2232698, n (%) SERPINE10 | 0 | 348 (98.9) | 72 (98.6) | 276 (98.9) | |
| | 1 | 4 (1.1) | 1 (1.4) | 3 (1.1) | 1.000 |
| (*) No carriers of the rs118203906 (Factor V Cambridge) and rs118203905 (Factor V Hong Kong) genetic variants were found in the cohort (nor in the VTE cases or in the non-VTE controls). | | | | | |

*VTE, mortality rates and ICU admission*

Table 7 shows the mortality rates recorded at 30 and 90 days after hospital admission, as well as admission to the ICU. Briefly, of the total 352 patients, 57 (16.2%) were admitted to ICU, and the global mortality rate at 90 days was 8.5% (30 patients). As expected, ICU admission was significantly associated with mortality at 30 and 90 days.

*Table 7. Mortality rates in the PRECIS_CQVID19 cohort and relationship with admission to the intensive care unit (ICU).*

| | PRECIS_CQVID19 (n=352) | Non-ICU (n=295) | ICU (n=57) | P |
|---|---|---|---|---|
| **Mortality at 30 days, n (%)** | 24 (6.8) | 13 (4.4) | 11 (19.3) | <0.001 |
| **Mortality at 90 days, n (%)** | 30 (8.5) | 16 (5.4) | 14 (24.6) | <0.001 |

Table 8 shows the associations between VTE status and 30 and 90 days mortality and ICU admission. Over 60% of the 73 patients who suffered a VTE event were admitted to the ICU, while <5% of the non-VTE controls received such attention.

*Table 8. Association between mortality rates and admission to the intensive care unit (ICU) with respect to venous thromboembolism (VTE) status.*

| | PRECIS_COVID19 (n=352) | Patients with VTE (n=73) | Non-VTE Controls (n=279) | P |
|---|---|---|---|---|
| **ICU, n (%)** | 57 (16.2) | 45 (61.6) | 12 (4.3) | <0.001 |
| **Mortality at 30 days, n (%)** | 24 (6.8) | 10 (13.7) | 14 (5.0) | 0.016 |
| **Mortality at 90 days, n (%)** | 30 (8.5) | 14 (19.2) | 16 (5.7) | <0.001 |

*Predictive capacity*

Table 9 shows the predictive capacity of each of the five models tested. Only the FVL+PT model showed no predictive capacity at all (not significant with respect to a random model of AUC 0.5). The TiC score, in contrast, showed excellent predictive capacity, with an AUC of 0.78 (95% confidence interval = 0.72-0.84), a sensitivity of 68.5%, and a specificity of 76.7%. Patients identified as being at high risk of suffering a VTE by the TiC score had an associated OR of 7.16. The accuracy measures shown in Table 7 for this model reveal a number needed to treat (NNT) of 2.3.

*Table 9: Capacity of the tested models to predict venous thromboembolic events (VTE). 'p'refers to the significance against a random model of area under the ROC curve (AUC) of 0.5. Numbers in parentheses are 95% confidence intervals.*

| | TiC | GRS | CRS | FVL+PT | FVL+PT+CRS |
|---|---|---|---|---|---|
| AUC | 0.781 [0.72 - 0.84] | 0.615 [0.54-0.69] | 0.756 [0.70-0.82] | 0.506 [0.43-0.58] | 0.757 [0.70 - 0.82] |
| P | <0.001 | 0.002 | <0.001 | 0.869 | <0.001 |
| Sensitivity | 68.5 [56.6 - 78.9] 50 / 73 | 52.1 [40.0 - 63.9] 38 / 73 | 71.2 [59.4 - 81.2] 52/73 | - | 65.8 [53.7 - 76.5] 48/73 |
| Specificity | 76.7 [71.3 - 81.5] 214 / 279 | 72.0 [66.4 - 72.2] 201 / 279 | 62.7 [56.8 - 68.4] 175 / 279 | - | 74.9 [69.4 - 79.9] 209/279 |
| PPV | 43.5 [34.3 - 53.0] 50 / 115 | 32.8 [24.3 - 42.1] 38 / 116 | 33.3 [26.0 - 41.3] 52 / 156 | - | 40.7 [31.7 - 50.1] 48 / 118 |
| NPV | 90.3 [85.8 - 93.7] 214 / 237 | 85.2 [80.0 - 89.5] 201 / 236 | 89.3 [84.1 - 93.2] 175 / 196 | - | 89.3 [84.6 - 93.0] 209 / 234 |
| LR+ | 2.94 | 1.87 | 1.91 | - | 2.63 |
| LR- | 0.41 | 0.67 | 0.46 | - | 0.46 |
| OR | 7.16 [4.06 - 12.61] | 2.80 [1.65 - 4.75] | 4.17 [2.38 - 7.31] | - | 5.73 [3.29 - 9.79] |

*TiC: Thrombo inCode score; GRS: genetic risk score; CRS: clinical risk score; FVL+PT: Factor V Leiden and Prothrombin mutations; FVL+PT+CRS: Factor V Leiden and Prothrombin mutations plus clinical risk score; PPV = positive predictive value; NPV = negative predictive value; LR = likelihood ratio; OR = odds ratio*

*Agreement between clinical and genetic risk scores*

The estimates returned by the CRS and GRS models showed no correlation ($\beta$=0.039; p=0.6).

*Discussion*

The impact of VTE on patients with COVID-19 could be drastically reduced if it were possible to assess each patient's risk of suffering an event. Ideally this would be determined at the time of hospital admission; the most appropriate prophylactic therapy could then be administered. With an AUC of 0.78 and promising predictive values, the TiC score is an excellent predictor of this risk. It should also be underscored that the TiC model is easy to use; it takes into account only twelve genetic variants plus clinical variables usually included in patient's records.

The incidence of VTE amongst the present cohort of patients, and its significant relationship with both ICU admission and mortality rates, are in agreement with results from previous reports[1,3,5,10].

While all patients received prophylactic anticoagulation treatment at admission, the results of clinical trials and epidemiological studies on such treatment are controversial. For example, a recent multicentre study[27] that examined the effect of intermediate vs. standard prophylactic doses in critically ill patients, reported no significant reduction in thrombotic complications, mortality, or poor outcome rates for the higher, intermediate dose regimen. Indeed, despite the inclusion of prophylactic measures in the second wave, no reduction in the overall incidence of VTE was observed in patients with COVID-19 compared to the first wave[43]. Therefore, the impact of VTE observed in the present cohort, despite the use of prophylactic treatment, is in agreement with these previous findings, and reinforces the need to improve prophylactic strategies. Higher doses still might prevent VTEs more effectively, but further research needs to be done in this area.

Driven by this need, this work assessed different scores for assessing VTE risk in patients with COVID-19. To date, most studies have aimed to establish prophylactic doses depending on patient clinical status. In addition, some authors have examined predictive models that take into account clinical and laboratory variables related to VTE[44-46]. However, to our knowledge, and despite the strong genetic component of VTE[47], no previous study has examined the intrinsic risk of patients suffering a VTE event.

The classical FVL + PT model had no capacity to predict a VTE event at all. While both genetic mutations contemplated imply a higher risk of VTE, their low frequency in the general population render them poor markers for predicting such events. Similar results have been reported previously[33,37]. The TiC score, which takes into account twelve genetic variants and five clinical variables from the original model, showed the best predictive capacity. This returned an AUC of 0.78, an OR of 7.16, and an NNT of 2.3. The genetic risk variants included in the TiC score have known functional consequences on the coagulation pathway[48-53], and the link between these variants and VTE has been established in different genome-wide association studies[54,55]. In addition, the useful predictive capacity of the GRS included in the TiC score has been validated in independent populations[3a,34]. Furthermore, it should be noted that one of the risk variants assessed is the A1 allele or haplotype for ABO blood type; the risk of VTE for A1 allele carriers is estimated to be increased by ~1.8 fold[56], and has also been significantly associated with different indicators of COVID-19 severity[57].

Certain clinical variables are associated with an increased risk of VTE, and different studies report a better capacity to predict VTE when genetic and clinical variables are combined.[37,58] In agreement with this, the present results show that the best predictive capacity was obtained when taking both into account - i.e., when using the TiC score. The results also show that the correlation between the risk estimates provided by the CRS and GRS models alone was not significant (p>0.05). These models may, therefore, reflect different sources of risk.

The pathophysiology underlying the prothrombotic status of patients with COVID-19 suggests a range of inter-related pathogenic mechanisms to be at work, including complement activation, hypoxia, and the activation of the immune system, among others[59,60]. Certainly, pathogenic dysregulation could trigger a prothrombotic status, but the present study hypothesizes that each patient has an intrinsic risk of VTE - a risk that needs to be evaluated. The same is seen in patients with cancer; there is a risk associated with the disease itself (due, for example, to the involvement of inflammation or immunity pathways), but outcomes are also influenced by the patient's intrinsic risk[61]. Certainly, prothrombotic mutations increase the risk of VTE in patients with cancer [62]. Indeed, the genetic variants in the TiC model have been validated in such patients, and are of known use in predicting VTEs[63]. It thus seems logical that intrinsic risk needs to be taken into account in patients with COVID-19, as the present results confirm.

It is important to know the risk of VTE at the time of admission; this is the moment when the TiC score is of full clinical value. Although all patients were receiving prophylactic treatment, the TiC model showed that 68.5% of patients that ended up having a VTE event may have benefited from more intense prophylaxis than they received.

In conclusion, the present work shows that the TiC score is useful in identifying patients with COVID-19 at high risk of VTE. Assessing the risk of VTE with this score at the time of hospital admission might indicate the most appropriate steps to follow and a personalized treatment pathway, thus reducing the incidence of VTE in patients with COVID-19.

*References*

1. Malas MB, Naazie IN, Elsayed N, Mathlouthi A, Marmor R, Clary B. Thromboembolism risk of COVID-19 is high and associated with a higher risk of mortality: A systematic review and meta-analysis. EClinicalMedicine. 2020;29:100639. doi:10.1016/j.eclinm.2020.100639

2. Lu Y, Pan L, Zhang W-W, Cheng F, Hu S-S, Zhang X, Jiang H. A meta-analysis of the incidence of venous thromboembolic events and impact of anticoagulation on mortality in patients with COVID-19. Int J Infect Dis. 2020;100:34-41. doi:https://doi.org/10.1016/j.ijid.2020.08.023

3. Porfidia A, Valeriani E, Pola R, Porreca E, Rutjes AWS, Di Nisio M. Venous thromboembolism in patients with COVID-19: Systematic review and meta-analysis. Thromb Res. 2020;196:67-74. doi:https://doi.org/10.1016/j.thromres.2020.08.020

4. Kunutsor SK, Laukkanen JA. Incidence of venous and arterial thromboembolic complications in COVID-19: A systematic review and meta-analysis. Thromb Res. 2020;196:27-30. doi:10.1016/j.thromres.2020.08.022

5. Nopp S, Moik F, Jilma B, Pabinger I, Ay C. Risk of venous thromboembolism in patients with COVID-19: A systematic review and meta-analysis. Res Pract Thromb Haemost. 2020;4(7):1178-1191. doi:10.1002/rth2.12439

6. Di Minno A, Ambrosino P, Calcaterra I, Di Minno MND. COVID-19 and Venous Thromboembolism: A Meta-analysis of Literature Studies. Semin Thromb Hemost. 2020;46(7):763-771. doi:10.1055/s-0040-1715456

7. Al-Ani F, Chehade S, Lazo-Langner A. Thrombosis risk associated with COVID-19 infection. A scoping review. Thromb Res. 2020;192:152-160. doi:10.1016/j.thromres.2020.05.039

8. Jiménez D, Garcia-SanchezA, Rali P, Muriel A, Bikdeli B, Ruiz-Artacho P, Le Mao R, Rodriguez C, Hunt BJ, Monreal M. Incidence of VTE and Bleeding Among Hospitalized Patients With Coronavirus Disease 2019: A Systematic Review and Meta-analysis. Chest. 2021;159(3):1182-1196. doi:10.1016/j.chest.2020.11.005

9. Boonyawat K, Chantrathammachart P, Numthavaj P, Nanthatanti N, Phusanti S, Phuphuakrat A, Niparuck P, Angchaisuksiri P. Incidence of thromboembolism in patients with COVID-19: a systematic review and meta-analysis. Thromb J. 2020;18(1):34. doi:10.1186/s12959-020-00248-5

10. Chi G, Lee JJ, Jamil A, Gunnam V, Najafi H, Memar Montazerin S, Shojaei F, Marszalek J. Venous Thromboembolism among Hospitalized Patients with COVID-19 Undergoing Thromboprophylaxis: A Systematic Review and Meta-Analysis. J Clin Med. 2020;9(8). doi:10.3390/jcm9082489

11. Demelo-Rodriguez P, Cervilla-Muñoz E, Ordieres-Ortega L, Parra-Virto A, Toledano-Macías M, Toledo-Samaniego N, García-García A, García-Fernández-Bravo I, Ji Z, de-Miguel-Diez J, Alvarez-Sala-Walther LA, Del-Toro-Cervera J, Galeano-Valle F. Incidence of asymptomatic deep vein thrombosis in patients with COVID-19 pneumonia and elevated D-dimer levels. Thromb Res. 2020;192:23-26. doi:10.1016/j.thromres.2020.05.018

12. Santoliquido A, Porfidia A, Nesci A, De Matteis G, Marrone G, Porceddu E, Cammà G, Giarretta I, Fantoni M, Landi F, Gasbarrini A, Pola R, D'Alfonso ME, Lo Monaco MR. Incidence of deep vein thrombosis among non-ICU patients hospitalized for COVID-19 despite pharmacological thromboprophylaxis. J Thromb Haemost. 2020;18(9):2358-2363. doi:10.1111/jth.14992

13. Maatman TK, Jalali F, Feizpour C, Douglas All, McGuire SP, Kinnaman G, Hartwell JL, Maatman BT, Kreutz RP, Kapoor R, Rahman O, Zyromski NJ, Meagher AD. Routine Venous Thromboembolism Prophylaxis May Be Inadequate in the Hypercoagulable State of Severe Coronavirus Disease 2019. Crit Care Med. 2020;48(9). https://journals.lww.eom/ccmjournal/Fulltext/2020/09000/Routine_Venous_Thro mboembolism_Prophylaxis_May_Be.33.aspx

14. Grandmaison G, Andrey A, Périard D, Engelberger RP, Carrel G, Doll S, Dexpert J-B, Krieger C, Ksouri H, Hayoz D, Sridharan G. Systematic Screening for Venous Thromboembolic Events in COVID-19 Pneumonia. TH open companion J to Thromb Haemost. 2020;4(2):e113-e115. doi:10.1055/s-0040-1713167

15. Betoule A, Martinet C, Gasperini G, Muller P, Foucher S, Benner P, Renard A. Diagnosis of venous and arterial thromboembolic events in COVID-19 virus-infected patients. J Thromb Thrombolysis. 2020;50(2):302-304. doi:10.1007/s 1 1239-020-02163-y

16. Ierardi AM, Coppola A, Fusco S, Stellato E, Aliberti S, Andrisani MC, Vespro V, Arrichiello A, Panigada M, Monzani V, Grasselli G, Venturini M, Rehani B, Peyvandi F, Pesenti A, Blasi F, Carrafiello G. Early detection of deep vein thrombosis in patients with coronavirus disease 2019: who to screen and who not to with Doppler ultrasound? J Ultrasound. Published online August 2020:1-9. doi:10.1007/s40477-020-00515-1

17. Thachil J, Tang N, Gando S, Falanga A, Cattaneo M, Levi M, Clark C, Iba T. ISTH interim guidance on recognition and management of coagulopathy in COVID-19. J Thromb Haemost. 2020;18(5):1023-1026. doi:10.1111/jth.14810

18. Spyropoulos AC, Levy JH, Ageno W, Connors JM, Hunt BJ, Iba T, Levi M, Samama CM, Thachil J, Giannis D, Douketis JD. Scientific and Standardization Committee communication: Clinical guidance on the diagnosis, prevention, and treatment of venous thromboembolism in hospitalized patients with COVID-19. J Thromb Haemost. 2020;18(8):1859-1865. doi:10.1111/jth.14929

19. Cuker A, Tseng EK, Nieuwlaat R, Angchaisuksiri P, Blair C, Dane K, Davila J, DeSancho MT, Diuguid D, Griffin DO, Kahn SR, Klok FA, Lee AI, Neumann I, Pai A, Pai M, Righini M, Sanfilippo KM, Siegal D, Skara M, Touri K, A. Akl E, Bou Akl I, Boulos M, Brignardello-Petersen R, Charide R, Chan M, Dearness K, Darzi AJ, Kolb P, Colunga-Lozano LE, Mansour R, Morgano GP, Morsi RZ, Noori A, Piggott T, Qiu Y, Roldan Y, Schünemann F, Stevens A, Solo K, Ventresca M, Wiercioch W, Mustafa RA, Schünemann HJ. American Society of Hematology 2021 guidelines on the use of anticoagulation for thromboprophylaxis in patients with COVID-19. Blood Adv. 2021;5(3):872-888. doi:10.1182/bloodadvances.2020003763

20. Fraissé M, Logre E, Pajot O, Mentec H, Plantefève G, Contou D. Thrombotic and hemorrhagic events in critically ill COVID-19 patients: a French monocenter retrospective study. Crit Care. 2020;24(1):275. doi:10.1186/s13054-020-03025-y

21. Al-Samkari H, Karp Leaf RS, Dzik WH, Carlson JCT, Fogerty AE, Waheed A, Goodarzi K, Bendapudi PK, Bornikova L, Gupta S, Leaf DE, Kuter DJ, Rosovsky RP. COVID-19 and coagulation: bleeding and thrombotic manifestations of SARS-CoV-2 infection. Blood. 2020;136(4):489-500. doi:10.1182/blood.2020006520

22. Patell R, Bogue T, Bindal P, Koshy A, Merrill M, Aird WC, Bauer KA, Zwicker JI. Incidence of thrombosis and hemorrhage in hospitalized cancer patients with COVID-19. J Thromb Haemost. 2020;18(9):2349-2357. doi:https://doi.org/10.1111/jth.15018

23. Pesavento R, Ceccato D, Pasquetto G, Monticelli J, Leone L, Frigo A, Gorgi D, Postal A, Marchese GM, Cipriani A, Saller A, Sarais C, Criveller P, Gemelli M, Capone F, Fioretto P, Pagano C, Rossato M, Avogaro A, Simioni P, Prandoni P, Vettor R. The hazard of (sub)therapeutic doses of anticoagulants in non-critically ill patients with Covid-19: The Padua province experience. J Thromb Haemost. 2020;18(10):2629-2635. doi:https://doi.org/10.1111/jth.15022

24. Kessler C, Stricker H, Demundo D, Elzi L, Monotti R, Bianchi G, Llamas M, Spinedi L, Rossi D, Chiesa AF, Pagnamenta A, Conti M, Casso G, Stoira E, Valenti E, Colucci G, Stussi G, Gerber B, Previsdomini M. Bleeding prevalence in COVID-19 patients receiving intensive antithrombotic prophylaxis. J Thromb Thrombolysis. 2020;50(4):833-836. doi:10.1007/s11239-020-02244-y

25. Lynn L, Reyes JA, Hawkins K, Panda A, Linville L, Aldhahri W, Kango G, Shah S, Ayanian S, Teufel K. The effect of anticoagulation on clinical outcomes in novel Coronavirus (COVID-19) pneumonia in a U.S. cohort. Thromb Res. 2021;197:65-68. doi:10.1016/j.thromres.2020.10.031

26. Rosovsky RP, Sanfilippo KM, Wang TF, Rajan SK, Shah S, Martin KA, Ní Áinle F, Huisman M, Hunt BJ, Kahn SR, Kevane B, Lee AYY, McLintock C, Baumann Kreuziger L. Anticoagulation practice patterns in COVIDD19: A global survey. Res Pract Thromb Haemost. 2020;4(6):969-983. doi:10.1002/rth2.12414

27. Sadeghipour P, Talasaz AH, Rashidi F, Sharif-Kashani B, Beigmohammadi MT, Farrokhpour M, Sezavar SH, Payandemehr P, Dabbagh A, Moghadam KG, Jamalkhani S, Khalili H, Yadollahzadeh M, Riahi T, Rezaeifar P, Tahamtan O, Matin S, Abedini A, Lookzadeh S, Rahmani H, Zoghi E, Mohammadi K, Sadeghipour P, Abri H, Tabrizi S, Mousavian SM, Shahmirzaei S, Bakhshandeh H, Amin A, Rafiee F, Baghizadeh E, Mohebbi B, Parhizgar SE, Aliannejad R, Eslami V, Kashefizadeh A, Kakavand H, Hosseini SH, Shafaghi S, Ghazi SF, Najafi A, Jimenez D, Gupta A, Madhavan M V, Sethi SS, Parikh SA, Monreal M, Hadavand N, Hajighasemi A, Maleki M, Sadeghian S, Piazza G, Kirtane AJ, Van Tassell BW, Dobesh PP, Stone GW, Lip GYH, Krumholz HM, Goldhaber SZ, Bikdeli B. Effect of Intermediate-Dose vs Standard-Dose Prophylactic Anticoagulation on Thrombotic Events, Extracorporeal Membrane Oxygenation Treatment, or Mortality Among Patients With COVID-19 Admitted to the Intensive Care Unit: The INSPIRATION Randomized Clini. JAMA. 2021;325(16):1620-1630. doi:10.1001/jama.2021.4152

28. Talasaz AH, Sadeghipour P, Kakavand H, Aghakouchakzadeh M, Kordzadeh-Kermani E, Van Tassell BW, Gheymati A, Ariannejad H, Hosseini SH, Jamalkhani S, Sholzberg M, Monreal M, Jimenez D, Piazza G, Parikh SA, Kirtane AJ, Eikelboom JW, Connors JM, Hunt BJ, Konstantinides S V, Cushman M, Weitz JI, Stone GW, Krumholz HM, Lip GYH, GoldhaberSZ, Bikdeli B. Recent Randomized Trials of Antithrombotic Therapy for Patients With COVID-19: JACC State-of-the-Art Review. J Am Coll Cardiol. 2021;77(15):1903-1921. doi:https://doi.org/10.1016/j.jacc.2021.02.035

29. Righini M, Perrier A, De Moerloose P, Bounameaux H. D-Dimer for venous thromboembolism diagnosis: 20 years later. J Thromb Haemost. 2008;6(7):1059-1071. doi:10.1111/j.1538-7836.2008.02981.x

30. Rosendaal FR. Venous thrombosis: A multicausal disease. Lancet. 1999;353(9159):1167-1173. doi:10.1016/S0140-6736(98)10266-0

31. Morange PE, Suchon P, Trégouët DA. Genetics of venous thrombosis: Update in 2015. Thromb Haemost. 2015;114(5):910-919. doi:10.1160/TH 15-05-0410

32. Souto JC, Almasy L, Borrell M, Blanco-Vaca F, Mateo J, Soria JM, Coll I, Felices R, Stone W, Fontcuberta J, Blangero J. Genetic susceptibility to thrombosis and its relationship to physiological risk factors: the GAIT study. Genetic Analysis of Idiopathic Thrombophilia. Am J Hum Genet. 2000;67(6):1452-1459. doi:10.1086/316903

33. Soria JM, Morange PE, Vila J, Souto JC, Moyano M, Trégouët DA, Mateo J, Saut N, Salas E, Elosua R. Multilocus genetic risk scores for venous thromboembolism risk assessment. J Am Heart Assoc. 2014;3(5):1-10. doi:10.1161/JAHA.114.001060

34. McInnes G, Daneshjou R, Katsonis P, Lichtarge O, Srinivasan R, Rana S, Radivojac P, Mooney SD, Pagel KA, Stamboulian M, Jiang Y, Capriotti E, Wang Y, Bromberg Y, Bovo S, Savojardo C, Martelli PL, Casadio R, Pal LR, Moult J, Brenner SE, Altman R. Predicting venous thromboembolism risk from exomes in the Critical Assessment of Genome Interpretation (CAGI) challenges. Hum Mutat. 2019;40(9):1314-1320. doi:10.1002/humu.23825

35. Zermatten MG, Pantet O, Gomez F, Schneider A, Mean M, Mazzolai L, Hugli O, Bart P-A, Papadimitriou-Olivgeris M, Alberio L, initiative C-19 IC-C. Utility of D-dimers and intermediate-dose prophylaxis for venous thromboembolism in critically ill patients with COVID-19. Thromb Res. 2020;196:222-226. doi: 10.1016/j.thromres.2020.08.027

36. Choi JJ, Wehmeyer GT, Li HA, Alshak MN, Nahid M, Rajan M, Liu B, Schatoff EM, Elahjji R, Abdelghany Y, D'Angelo D, Crossman D, Evans AT, Steel P, Pinheiro LC, Goyal P, Safford MM, Mints G, DeSancho MT. D-dimer cut-off points and risk of venous thromboembolism in adult hospitalized patients with COVID-19. Thromb Res. 2020;196:318-321. doi:10.1016/j.thromres.2020.09.022

37. Bruzelius M, Bottai M, Sabater-Lleal M, Strawbridge RJ, Bergendal A, Silveira A, Sundström A, Kieler H, Hamsten A, Odeberg J. Predicting venous thrombosis in women using a combination of genetic markers and clinical risk factors. J Thromb Haemost. 2015;13(2):219-227. doi:10.1111/jth.12808

38. McDaid A, Logette E, Buchillier V, Muriset M, Suchon P, Pache TD, Tanackovic G, Kutalik Z, Michaud J. Risk prediction of developing venous thrombosis in combined oral contraceptive users. PLoS One. 2017;12(7):e0182041. doi: 10.1371/journal. pone. 0182041

39. Rosendaal FR. Causes of Venous Thrombosis. Deep Vein Thromb Pulm Embolism. 2009;14(Suppl 1):1-26. doi:10.1002/9780470745007.ch1

40. Hajian-Tilaki K. Receiver Operating Characteristic (ROC) Curve Analysis for Medical Diagnostic Test Evaluation. Casp J Intern Med. 2013;4(2):627-635.

41. Ruopp MD, Perkins NJ, Whitcomb BW, Schisterman EF. Youden Index and optimal cut-point estimated from observations affected by a lower limit of detection. Biom J. 2008;50(3):419-430. doi:10.1002/bimj.200710415

42. DeLong ER, DeLong DM, Clarke-Pearson DL. Comparing the areas under two or more correlated receiver operating characteristic curves: a nonparametric approach. Biometrics. 1988;44(3):837-845.

43. Kaptein FHJ, Stals MAM, Grootenboers M, Braken SJE, Burggraaf JLI, van Bussel BCT, Cannegieter SC, Ten Cate H, Endeman H, Gommers DAMPJ, van Guldener C, de Jonge E, Juffermans NP, Kant KM, Kevenaar ME, Koster S, Kroft LJM, Kruip MJHA, Leentjens J, Marechal C, Soei YL, Tjepkema L, Visser C, Klok FA, Huisman M V. Incidence of thrombotic complications and overall survival in hospitalized patients with COVID-19 in the second and first wave. Thromb Res. 2021;199:143-148. doi:10.1016/j.thromres.2020.12.019

44. Spyropoulos AC, Cohen SL, Gianos E, Kohn N, Giannis D, Chatterjee S, Goldin M, Lesser M, Coppa K, Hirsch JS, McGinn T, Barish MA, Group the C-19 C. Validation of the IMPROVE-DD risk assessment model for venous thromboembolism among hospitalized patients with COVID-19. Res Pract Thromb Haemost. 2021;5(2):296-300. doi:https://doi.org/10.1002/rth2.12486

45. Dujardin RWG, Hilderink BN, Haksteen WE, Middeldorp S, Vlaar APJ, Thachil J, Muller MCA, Juffermans NP. Biomarkers for the prediction of venous thromboembolism in critically ill COVID-19 patients. Thromb Res. 2020;196:308-312. doi:10.1016/j.thromres.2020.09.017

46. Kampouri E, Filippidis P, Viala B, Mean M, Pantet O, Desgranges F, Tschopp J, Regina J, Karachalias E, Bianchi C, Zermatten MG, Jaton K, Qanadli SD, Bart PA, Pagani J-L, Guery B, Alberio L, Papadimitriou-Olivgeris M, Reg-COVID Research Group. Predicting Venous Thromboembolic Events in Patients with Coronavirus Disease 2019 Requiring Hospitalization: an Observational Retrospective Study by the COVIDIC Initiative in a Swiss University Hospital. Su C-M, ed. Biomed Res Int. 2020;2020:9126148. doi:10.1155/2020/9126148

47. Delaneau O, Zagury JF, Marchini J. Improved whole-chromosome phasing for disease and population genetic studies. Nat Methods. 2013;10(1):5-6. doi: 10. 1038/nmeth.2307

48. O'Donnell J, Boulton FE, Manning RA, Laffan MA. Amount of H Antigen Expressed on Circulating von Willebrand Factor Is Modified by ABO Blood Group Genotype and Is a Major Determinant of Plasma von Willebrand Factor Antigen Levels. Arterioscler Thromb Vasc Biol. 2002;22(2):335-341. doi:10.1161/hq0202.103997

49. Corral J, Gonzalez-Conejero R, Soria JM, Gonzalez-Porras JR, Perez-Ceballos E, Lecumberri R, Roldan V, Souto JC, MiñanoA, Hernandez-Espinosa D, Alberca I, Fontcuberta J, Vicente V. A nonsense polymorphism in the protein Z-dependent protease inhibitor increases the risk for venous thrombosis. Blood. 2006; 108(1): 177-183. doi: 10.1182/blood-2005-08-3249

50. Poort SR, Rosendaal FR, Reitsma PH, Bertina RM. A common genetic variation in the 3'-untranslated region of the prothrombin gene is associated with elevated plasma prothrombin levels and an increase in venous thrombosis. Blood. 1996;88(10):3698-3703.

51. Bertina RM, Koeleman BP, Koster T, Rosendaal FR, Dirven RJ, de Ronde H, van der Velden PA, Reitsma PH. Mutation in blood coagulation factor V associated with resistance to activated protein C. Nature. 1994;369(6475):64-67. doi: 10.1038/369064a0

52. Van Hylckama Vlieg A, Komanasin N, Ariëns RAS, Poort SR, Grant PJ, Bertina RM, Rosendaal FR. Factor XIII Val34Leu polymorphism, factor XIII antigen levels and activity and the risk of deep venous thrombosis. Br J Haematol. 2002;119(1):169-175. doi: 10.1046/j.1365-2141.2002.03797.x

53. Kanaji T, Okamura T, Osaki K, Kuroiwa M, Shimoda K, Hamasaki N, Niho Y. A common genetic polymorphism (46 C to T substitution) in the 5'-untranslated region of the coagulation factor XII gene is associated with low translation efficiency and decrease in plasma factor XII level. Blood. 1998;91(6):2010-2014.

54. Klarin D, Busenkell E, Judy R, Lynch J, Levin M, Haessler J, Aragam K, Chaffin M, Haas M, Lindström S, Assimes TL, Huang J, Min Lee K, Shao Q, Huffman JE, Kabrhel C, Huang Y, Sun Y V, Vujkovic M, Saleheen D, Miller DR, Reaven P, DuVall S, Boden WE, Pyarajan S, Reiner AP, Trégouët D-A, Henke P, Kooperberg C, Gaziano JM, Concato J, Rader DJ, Cho K, Chang K-M, Wilson PWF, Smith NL, O'Donnell CJ, Tsao PS, Kathiresan S, Obi A, Damrauer SM, Natarajan P, Consortium I, Program VAMV. Genome-wide association analysis of venous thromboembolism identifies new risk loci and genetic overlap with arterial vascular disease. Nat Genet. 2019;51(11):1574-1579. doi:10.1038/s41588-019-0519-3

55. Lindström S, Wang L, Smith EN, Gordon W, van Hylckama Vlieg A, de Andrade M, Brody JA, Pattee JW, Haessler J, Brumpton BM, Chasman DI, Suchon P, Chen M-H, Turman C, Germain M, Wiggins KL, MacDonald J, Braekkan SK, Armasu SM, Pankratz N, Jackson RD, Nielsen JB, Giulianini F, Puurunen MK, Ibrahim M, Heckbert SR, Damrauer SM, Natarajan P, Klarin D, Program TMV, de Vries PS, Sabater-Lleal M, Huffman JE, Group TCHW, Bammler TK, Frazer KA, McCauley BM, Taylor K, Pankow JS, Reiner AP, Gabrielsen ME, Deleuze J-F, O'Donnell CJ, Kim J, McKnight B, Kraft P, Hansen J-B, Rosendaal FR, Heit JA, Psaty BM, Tang W, Kooperberg C, Hveem K, Ridker PM, Morange P-E, Johnson AD, Kabrhel C, Trégouët D-A, Smith NL, Consortium on behalf of the I. Genomic and transcriptomic association studies identify 16 novel susceptibility loci for venous thromboembolism. Blood. 2019;134(19):1645-1657. doi: 10.1182/blood.2019000435

56. Goumidi L, Thibord F, Wiggins KL, Li-Gao R, Brown MR, van Hylckama Vlieg A, Souto J-C, Soria J-M, Ibrahim-Kosta M, Saut N, Daian D, Olaso R, Amouyel P, Debette S, Boland A, Bailly P, Morrison AC, Mook-Kanamori DO, Deleuze J-F, Johnson A, de Vries PS, Sabater-Lleal M, Chiaroni J, Smith NL, Rosendaal FR, Chasman DI, Trégouët D-A, Morange P-E. Association between ABO haplotypes and the risk of venous thrombosis: impact on disease risk estimation. Blood. 2021;137(17):2394-2402. doi: 10.1182/blood.2020008997

57. Hoiland RL, Fergusson NA, Mitra AR, Griesdale DEG, Devine D V, Stukas S, Cooper J, Thiara S, Foster D, Chen LYC, Lee AYY, Conway EM, Wellington CL, Sekhon MS. The association of ABO blood group with indices of disease severity and multiorgan dysfunction in COVID-19. Blood Adv. 2020;4(20):4981-4989. doi:10.1182/bloodadvances.2020002623

58. De Haan HG, Bezemer ID, Doggen CJM, Cessie S Le, Reitsma PH, Arellano AR, Tong CH, Devlin JJ, Bare LA, Rosendaal FR, Vossen CY. Multiple SNP testing improves risk prediction of first venous thrombosis. Blood. 2012;120(3):656-663. doi:10.1182/blood-2011 -12-397752

59. Voicu S, Ketfi C, Stépanian A, Chousterman BG, Mohamedi N, Siguret V, Mebazaa A, Megarbane B, Bonnin P. Pathophysiological Processes Underlying the High Prevalence of Deep Vein Thrombosis in Critically III COVID-19 Patients. Front Physiol. 2020;11:608788. doi:10.3389/fphys.2020.608788

60. Loo J, Spittle DA, Newnham M. COVID-19, immunothrombosis and venous thromboembolism: biological mechanisms. Thorax. 2021;76(4):412 LP - 420. doi:10.1136/thoraxjnl-2020-216243

61. Abdol Razak NB, Jones G, Bhandari M, Berndt MC, Metharom P. Cancer-Associated Thrombosis: An Overview of Mechanisms, Risk Factors, and Treatment. Cancers (Basel). 2018;10(10):380. doi:10.3390/cancers10100380

62. Pabinger I, Ay C, Dunkler D, Thaler J, Reitter E-M, Marosi C, Zielinski C, Mannhalter C. Factor V Leiden mutation increases the risk for venous thromboembolism in cancer patients - results from the Vienna Cancer And Thrombosis Study (CATS). J Thromb Haemost. 2015;13(1):17-22. doi:10.1111/jth.12778

63. Muñoz Martin AJ, Ortega I, Font C, Pachón V, Castellón V, Martínez-Marín V, Salgado M, Martinez E, Calzas J, Rupérez A, Souto JC, Martin M, Salas E, Soria JM. Multivariable clinical-genetic risk model for predicting venous thromboembolic events in patients with cancer. Br J Cancer. 2018;118(8):1056-1061. doi:10.1038/s41416-018-0027-8

**Claims**

1. A method for the thromboembolic event or disease risk assessment in a subject suffering from COVID-19, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548)), factor XIII Val34Leu (rs5985), factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or ABO blood group rs8176743, and ABO blood group rs8176750, which is indicative of a risk of having or developing a thromboembolic event or disease.

2. A method for predicting the severity of disease in a subject suffering from COVID-19, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or ABO blood group rs8176743, and ABO blood group rs8176750, which is indicative of a risk of developing severe disease.

3. The method of claim 2, wherein the severe disease comprises admission of the subject to an intensive care unit and/or mortality.

4. The method of any one preceding claim, wherein the subject is previously classified as being vulnerable to COVID-19.

5. A method for identifying a subject suffering from COVID-19 in need of anticoagulant and/or antithrombotic therapy, prophylactic antithrombotic and/or anticoagulant therapy, or a more aggressive program thereof, comprising the steps of determining in a sample isolated from said subject the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), Factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176749 or rs8176743, and ABO blood group rs8176750, which is indicative of being in need of anticoagulant and/or antithrombotic therapy, prophylactic antithrombotic and/or anticoagulant therapy, or a more aggressive program thereof.

6. The method of any one preceding claim, further comprising determining one or more risk factor(s) selected from the group consisting of age, sex, body mass index, smoking status, and diabetes mellitus, optionally wherein each of age, sex, body mass index, smoking status, and diabetes mellitus are determined.

7. The method of any one preceding claim, wherein the sample is an oral tissue sample, scraping, or wash or a biological fluid sample, preferably saliva, urine or blood.

8. The method of any one preceding claim, wherein the presence or absence of the polymorphism(s) is identified by allele-specific PCR.

9. A method of determining the probability of an individual suffering from COVID-19 of presenting a thromboembolic disease or event based on 1 to n variables including the presence or absence of the following polymorphisms or respective SNPs in strong linkage disequilibrium with said polymorphisms: Serpin C1 (antithrombin) Ala384Ser (Cambridge II) (rs121909548), factor XIII Val34Leu (rs5985), Factor II (prothrombin) G20210A (rs1799963), and optionally any one or more of Serpin A10 (protein Z inhibitor) Arg67Stop (rs2232698), factor XII C46T (rs1801020), factor V Leiden Arg506Gln (rs6025), factor V Cambridge Arg306Thr (rs118203906), factor V Hong Kong Arg306Gly (rs118203905), ABO blood group rs8176719, ABO blood group rs7853989, ABO blood group rs8176743 or rs8176749, and ABO blood group rs8176750 using the formula:

$$\text{Probability } (Y=1|x_1,\ldots, x_n) = 1 / 1 + \exp (\beta_0 + \beta_1 x_1 + \ldots + \beta_n x_n),$$

wherein:

- Probability $(Y = 1 \mid x_1, \ldots, x_n)$ = probability of presenting a thrombosis in a particular individual with concrete and measurable characteristics in a number of variables 1, ...., n, wherein said probability could range between 0 and 1;
- Exp = exponential natural base;
- $\beta_0$ = coefficient that defines the risk (the probability) of thrombosis non related with the variables 1 to n, wherein said coefficient can take a value from $-\infty$ to $+\infty$ and is calculated as the natural logarithm of the incidence of venous thrombosis in the population;
- $\beta_1$ = regression coefficient that expresses the risk (higher or lower) to present thrombosis associated with the value/presence of the predictor variable $x_1$, wherein said coefficient can take a value from $-\infty$ to $+\infty$;
- $x_1$ = value taken by the predictor variable $x_1$ in an individual, wherein the range of possible values depends on the variable;
- $\beta_n$ = regression coefficient that expresses the risk (higher or lower) to present thrombosis associated with the value/presence of the predictor variable $x_n$, wherein said coefficient can take a value from $-\infty$ to $+\infty$;
- $x_n$ = value taken by the predictor variable $x_n$ in an individual, wherein the range of possible values depends on the variable.

10. The method of any one preceding claim, wherein the thromboembolic event or disease is selected from the group of deep vein thrombosis, pulmonary embolism or a combination thereof.

11. The method of any one preceding claim, wherein the subject is admitted to hospital with COVID-19, and optionally administered prophylactic anticoagulation therapy, optionally wherein the prophylactic anticoagulation therapy is administered on admission to hospital.

12. The method of any one preceding claim, wherein a strong linkage disequilibrium is one with an $r^2$ value of more than 0.7, more than 0.8, or more than 0.9, wherein $r^2$ is defined as follows:

$$r^2(p_a, p_b, p_{ab}) = \frac{(p_{ab} - p_a p_b)^2}{p_a(1 - p_a) p_b(1 - p_b)},$$

where $p_{ab}$ is the frequency of haplotypes having allele $\alpha$ at locus 1 and allele $b$ at locus 2

**13.** A computer program or a computer-readable media containing means for carrying out the method as defined in any one of the preceding claims.

Figure 1

Europäisches
Patentamt

European
Patent Office

Office européen
des brevets

# EUROPEAN SEARCH REPORT

**Application Number**

EP 21 38 3122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SORIA JOSÉ MANUEL ET AL: "Multilocus Genetic Risk Scores for Venous Thromboembolism Risk Assessment", JOURNAL OF THE AMERICAN HEART ASSOCIATION, vol. 3, no. 5, 16 September 2014 (2014-09-16), XP055919985, ISSN: 2047-9980, DOI: 10.1161/JAHA.114.001060 | 1,6,8, 10,13 | INV. C12Q1/70 C12Q1/6883 |
| Y | * the whole document * <br> * abstract, Table 1 * <br> ----- | 2-5,7,9, 11,12 | |
| X | EP 2 535 424 A1 (GENDIAG EXE SL) 19 December 2012 (2012-12-19) | 1,6,7, 10,13 | |
| Y | * the whole document * <br> * para. 58, 63, 82, claim 1 * <br> ----- | 2-5,8,9, 11,12 | |
| Y | MORENA-BARRIO MARIA EUGENIA ET AL: "A pilot study on the impact of congenital thrombophilia in COVID-19", EUROPEAN JOURNAL OF CLINICAL INVESTIGATION, vol. 51, no. 5, 25 May 2021 (2021-05-25), XP055920121, GB ISSN: 0014-2972, DOI: 10.1111/eci.13546 Retrieved from the Internet: URL:https://onlinelibrary.wiley.com/doi/fu ll-xml/10.1111/eci.13546> * the whole document * * abstract, Table 1 * ----- | 1-13 | TECHNICAL FIELDS SEARCHED (IPC) <br><br> C12Q |
| Y | WO 2018/215590 A1 (GENDIAG EXE SL [ES]) 29 November 2018 (2018-11-29) * the whole document * * p. 8, claim 10 * ----- | 1-13 | |

-/--

The present search report has been drawn up for all claims

| Place of search | Date of completion of the search | Examiner |
|---|---|---|
| Munich | 23 May 2022 | Sauer, Tincuta |

EPO FORM 1503 03.82 (P04C01)

Europäisches
Patentamt

European
Patent Office

Office européen
des brevets

# EUROPEAN SEARCH REPORT

Application Number

EP 21 38 3122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2019/154996 A1 (GENINCODE UK LTD [GB]) 15 August 2019 (2019-08-15) * the whole document * * claim 8 * ----- | 1-13 | |

TECHNICAL FIELDS
SEARCHED      (IPC)

The present search report has been drawn up for all claims

| Place of search | Date of completion of the search | Examiner |
|---|---|---|
| Munich | 23 May 2022 | Sauer, Tincuta |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

............................................................................
& : member of the same patent family, corresponding
　　document

EPO FORM 1503 03.82 (P04C01)

page 2 of 2

## ANNEX TO THE EUROPEAN SEARCH REPORT
## ON EUROPEAN PATENT APPLICATION NO.

EP 21 38 3122

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2022

| Patent document cited in search report | | Publication date | Patent family member(s) | | Publication date |
|---|---|---|---|---|---|
| EP 2535424 | A1 | 19-12-2012 | AR | 086734 A1 | 22-01-2014 |
| | | | AU | 2012269112 A1 | 30-01-2014 |
| | | | BR | 112013032352 A2 | 30-10-2018 |
| | | | CA | 2839368 A1 | 20-12-2012 |
| | | | CL | 2013003605 A1 | 08-08-2014 |
| | | | DK | 2535424 T3 | 14-09-2015 |
| | | | DK | 2721172 T3 | 19-02-2018 |
| | | | DO | P2013000296 A | 31-03-2014 |
| | | | EP | 2535424 A1 | 19-12-2012 |
| | | | EP | 2721172 A2 | 23-04-2014 |
| | | | EP | 2799556 A2 | 05-11-2014 |
| | | | ES | 2547075 T3 | 01-10-2015 |
| | | | ES | 2663943 T3 | 17-04-2018 |
| | | | MX | 349347 B | 25-07-2017 |
| | | | MX | 360663 B | 13-11-2018 |
| | | | NO | 2721172 T3 | 09-06-2018 |
| | | | PE | 20141069 A1 | 06-09-2014 |
| | | | PT | 2535424 E | 29-10-2015 |
| | | | PT | 2721172 T | 21-02-2018 |
| | | | RU | 2014101169 A | 27-07-2015 |
| | | | US | 2014221230 A1 | 07-08-2014 |
| | | | US | 2016102355 A1 | 14-04-2016 |
| | | | WO | 2012171949 A2 | 20-12-2012 |
| WO 2018215590 | A1 | 29-11-2018 | EP | 3631016 A1 | 08-04-2020 |
| | | | WO | 2018215590 A1 | 29-11-2018 |
| WO 2019154996 | A1 | 15-08-2019 | CA | 3090339 A1 | 15-08-2019 |
| | | | EP | 3749783 A1 | 16-12-2020 |
| | | | JP | 2021513372 A | 27-05-2021 |
| | | | WO | 2019154996 A1 | 15-08-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6159693 A
- US 4683195 A
- US 4683202 A
- US 4800159 A
- US 4883750 A

### Non-patent literature cited in the description

- **HILL ; ROBERTSON.** *Theor Appl Genetics,* 1968, vol. 38, 226-231
- **BENTON ; DAVIS.** *Science,* 1977, vol. 196, 180
- **GRUNSTEIN ; HOGNESS.** *Proc. Natl. Acad. Sci., USA,* 1975, vol. 72, 3961
- **AUSUBEL et al.** Current Protocols in Molecular Biology. Wiley Interscience, 2001
- **BERGER ; KIMMEL.** Guide to Molecular Cloning Techniques. Academic Press, 1987
- **SAMBROOK et al.** Molecular Cloning: A Laboratory Manual. Cold Spring Harbor Laboratory Press, 1989
- **MALAS MB ; NAAZIE IN ; ELSAYED N ; MATHLOUTHI A ; MARMOR R ; CLARY B.** Thromboembolism risk of COVID-19 is high and associated with a higher risk of mortality: A systematic review and meta-analysis. *EClinicalMedicine,* 2020, vol. 29, 100639
- **LU Y ; PAN L ; ZHANG W-W ; CHENG F ; HU S-S ; ZHANG X ; JIANG H.** A meta-analysis of the incidence of venous thromboembolic events and impact of anticoagulation on mortality in patients with COVID-19. *Int J Infect Dis.,* 2020, vol. 100, 34-41, https://doi.org/10.1016/j.ijid.2020.08.023
- **PORFIDIA A ; VALERIANI E ; POLA R ; PORRECA E ; RUTJES AWS ; DI NISIO M.** Venous thromboembolism in patients with COVID-19: Systematic review and meta-analysis. *Thromb Res,* 2020, vol. 196, 67-74, https://doi.org/10.1016/j.thromres.2020.08.020
- **KUNUTSOR SK ; LAUKKANEN JA.** Incidence of venous and arterial thromboembolic complications in COVID-19: A systematic review and meta-analysis. *Thromb Res,* 2020, vol. 196, 27-30
- **NOPP S ; MOIK F ; JILMA B ; PABINGER I ; AY C.** Risk of venous thromboembolism in patients with COVID-19: A systematic review and meta-analysis. *Res Pract Thromb Haemost,* 2020, vol. 4 (7), 1178-1191
- **DI MINNO A ; AMBROSINO P ; CALCATERRA I ; DI MINNO MND.** COVID-19 and Venous Thromboembolism: A Meta-analysis of Literature Studies. *Semin Thromb Hemost,* 2020, vol. 46 (7), 763-771
- **AL-ANI F ; CHEHADE S ; LAZO-LANGNER A.** Thrombosis risk associated with COVID-19 infection. A scoping review. *Thromb Res,* 2020, vol. 192, 152-160
- **JIMÉNEZ D ; GARCIA-SANCHEZA ; RALI P ; MURIEL A ; BIKDELI B ; RUIZ-ARTACHO P ; LE MAO R ; RODRIGUEZ C ; HUNT BJ ; MONREAL M.** Incidence of VTE and Bleeding Among Hospitalized Patients With Coronavirus Disease 2019: A Systematic Review and Meta-analysis. *Chest,* 2021, vol. 159 (3), 1182-1196
- **BOONYAWAT K ; CHANTRATHAMMACHART P ; NUMTHAVAJ P ; NANTHATANTI N ; PHUSANTI S ; PHUPHUAKRAT A ; NIPARUCK P ; ANGCHAISUKSIRI P.** Incidence of thromboembolism in patients with COVID-19: a systematic review and meta-analysis. *Thromb J,* 2020, vol. 18 (1), 34
- **CHI G ; LEE JJ ; JAMIL A ; GUNNAM V ; NAJAFI H ; MEMAR MONTAZERIN S ; SHOJAEI F ; MARSZALEK J.** Venous Thromboembolism among Hospitalized Patients with COVID-19 Undergoing Thromboprophylaxis: A Systematic Review and Meta-Analysis. *J Clin Med.,* 2020, vol. 9 (8
- **DEMELO-RODRIGUEZ P ; CERVILLA-MUÑOZ E ; ORDIERES-ORTEGA L ; PARRA-VIRTO A ; TOLEDANO-MACÍAS M ; TOLEDO-SAMANIEGO N ; GARCÍA-GARCÍA A ; GARCÍA-FERNÁNDEZ-BRAVO I ; JI Z, DE-MIGUEL-DIEZ J ; ALVAREZ-SALA-WALTHER LA.** Incidence of asymptomatic deep vein thrombosis in patients with COVID-19 pneumonia and elevated D-dimer levels. *Thromb Res,* 2020, vol. 192, 23-26

- **SANTOLIQUIDO A ; PORFIDIA A ; NESCI A ; DE MATTEIS G ; MARRONE G ; PORCEDDU E ; CAMMÀ G ; GIARRETTA I ; FANTONI M ; LANDI F.** Incidence of deep vein thrombosis among non-ICU patients hospitalized for COVID-19 despite pharmacological thromboprophylaxis. *J Thromb Haemost,* 2020, vol. 18 (9), 2358-2363
- **MAATMAN TK ; JALALI F ; FEIZPOUR C ; DOUGLAS ALL ; MCGUIRE SP ; KINNAMAN G ; HARTWELL JL ; MAATMAN BT ; KREUTZ RP ; KAPOOR R.** Routine Venous Thromboembolism Prophylaxis May Be Inadequate in the Hypercoagulable State of Severe Coronavirus Disease. *Crit Care Med,* 2019, vol. 48 (9, https://journals.lww.eom/ccmjournal/Fulltext/2020/09000/Routine_Venous_Thro mboembolism_Prophylaxis_May_Be.33.aspx
- **GRANDMAISON G ; ANDREY A ; PÉRIARD D ; ENGELBERGER RP ; CARREL G ; DOLL S ; DEXPERT J-B ; KRIEGER C ; KSOURI H ; HAYOZ D.** Systematic Screening for Venous Thromboembolic Events in COVID-19 Pneumonia. *TH open companion J to Thromb Haemost,* 2020, vol. 4 (2), e113-e115
- **BETOULE A ; MARTINET C ; GASPERINI G ; MULLER P ; FOUCHER S ; BENNER P ; RENARD A.** Diagnosis of venous and arterial thromboembolic events in COVID-19 virus-infected patients. *J Thromb Thrombolysis,* 2020, vol. 50 (2), 302-304
- **LERARDI AM ; COPPOLA A ; FUSCO S ; STELLATO E ; ALIBERTI S ; ANDRISANI MC ; VESPRO V ; ARRICHIELLO A ; PANIGADA M ; MONZANI V.** Early detection of deep vein thrombosis in patients with coronavirus disease 2019: who to screen and who not to with Doppler ultrasound?. *J Ultrasound,* August 2020, 1-9
- **THACHIL J ; TANG N ; GANDO S ; FALANGA A ; CATTANEO M ; LEVI M ; CLARK C ; IBA T.** ISTH interim guidance on recognition and management of coagulopathy in COVID-19. *J Thromb Haemost,* 2020, vol. 18 (5), 1023-1026
- **SPYROPOULOS AC ; LEVY JH ; AGENO W ; CONNORS JM ; HUNT BJ ; IBA T ; LEVI M ; SAMAMA CM ; THACHIL J ; GIANNIS D.** Scientific and Standardization Committee communication: Clinical guidance on the diagnosis, prevention, and treatment of venous thromboembolism in hospitalized patients with COVID-19. *J Thromb Haemost.,* 2020, vol. 18 (8), 1859-1865
- **CUKER A ; TSENG EK ; NIEUWLAAT R ; ANGCHAISUKSIRI P ; BLAIR C ; DANE K ; DAVILA J ; DESANCHO MT ; DIUGUID D ; GRIFFIN DO.** American Society of Hematology 2021 guidelines on the use of anticoagulation for thromboprophylaxis in patients with COVID-19. *Blood Adv,* 2021, vol. 5 (3), 872-888
- **FRAISSÉ M ; LOGRE E ; PAJOT O ; MENTEC H ; PIANTEFÈVE G ; CONTOU D.** Thrombotic and hemorrhagic events in critically ill COVID-19 patients: a French monocenter retrospective study. *Crit Care,* 2020, vol. 24 (1), 275
- **AL-SAMKARI H ; KARP LEAF RS ; DZIK WH ; CARLSON JCT ; FOGERTY AE ; WAHEED A ; GOODARZI K ; BENDAPUDI PK ; BORNIKOVA L ; GUPTA S.** COVID-19 and coagulation: bleeding and thrombotic manifestations of SARS-CoV-2 infection. *Blood,* 2020, vol. 136 (4), 489-500
- **PATELL R ; BOGUE T ; BINDAL P ; KOSHY A ; MERRILL M ; AIRD WC ; BAUER KA ; ZWICKER JI.** Incidence of thrombosis and hemorrhage in hospitalized cancer patients with COVID-19. *J Thromb Haemost,* 2020, vol. 18 (9), 2349-2357, https://doi.org/10.1111/jth.15018
- **PESAVENTO R ; CECCATO D ; PASQUETTO G ; MONTICELLI J ; LEONE L ; FRIGO A ; GORGI D ; POSTAL A ; MARCHESE GM ; CIPRIANI A.** The hazard of (sub)therapeutic doses of anticoagulants in non-critically ill patients with Covid-19: The Padua province experience. *J Thromb Haemost,* 2020, vol. 18 (10), 2629-2635, https://doi.org/10.1111/jth.15022
- **KESSLER C ; STRICKER H ; DEMUNDO D ; ELZI L ; MONOTTI R ; BIANCHI G ; LLAMAS M ; SPINEDI L ; ROSSI D ; CHIESA AF.** Previsdomini M. Bleeding prevalence in COVID-19 patients receiving intensive antithrombotic prophylaxis. *J Thromb Thrombolysis,* 2020, vol. 50 (4), 833-836
- **LYNN L ; REYES JA ; HAWKINS K ; PANDA A ; LINVILLE L ; ALDHAHRI W ; KANGO G ; SHAH S ; AYANIAN S ; TEUFEL K.** The effect of anticoagulation on clinical outcomes in novel Coronavirus (COVID-19) pneumonia in a U.S. cohort. *Thromb Res.,* 2021, vol. 197, 65-68
- **HUISMAN M ; HUNT BJ ; KAHN SR ; KEVANE B ; LEE AYY ; MCLINTOCK C ; BAUMANN KREUZIGER L.** Anticoagulation practice patterns in COVIDD19: A global survey. *Res Pract Thromb Haemost,* 2020, vol. 4 (6), 969-983
- **SADEGHIPOUR P ; TALASAZ AH ; RASHIDI F ; SHARIF-KASHANI B ; BEIGMOHAMMADI MT ; FARROKHPOUR M ; SEZAVAR SH ; PAYANDEMEHR P ; DABBAGH A ; MOGHADAM KG.** Effect of Intermediate-Dose vs Standard-Dose Prophylactic Anticoagulation on Thrombotic Events, Extracorporeal Membrane Oxygenation Treatment, or Mortality Among Patients With COVID-19 Admitted to the Intensive Care Unit: The INSPIRATION Randomized Clini. *JAMA,* 2021, vol. 325 (16), 1620-1630

- **TALASAZ AH ; SADEGHIPOUR P ; KAKAVAND H ; AGHAKOUCHAKZADEH M ; KORDZA-DEH-KERMANI E ; VAN TASSELL BW ; GHEYMA-TI A ; ARIANNEJAD H ; HOSSEINI SH ; JAMAL-KHANI S.** Recent Randomized Trials of Antithrombotic Therapy for Patients With COVID-19: JACC State-of-the-Art Review. *J Am Coll Cardiol,* 2021, vol. 77 (15), 1903-1921, https://doi.org/10.1016/j.jacc.2021.02.035
- **RIGHINI M ; PERRIER A ; DE MOERLOOSE P ; BOUNAMEAUX H.** D-Dimer for venous thromboembolism diagnosis: 20 years later. *J Thromb Haemost,* 2008, vol. 6 (7), 1059-1071
- **ROSENDAAL FR.** Venous thrombosis: A multicausal disease. *Lancet,* 1999, vol. 353 (9159), 1167-1173
- **MORANGE PE ; SUCHON P ; TRÉGOUËT DA.** Genetics of venous thrombosis: Update in 2015. *Thromb Haemost,* 2015, vol. 114 (5), 910-919
- **SOUTO JC ; ALMASY L ; BORRELL M ; BLAN-CO-VACA F ; MATEO J ; SORIA JM ; COLL I ; FE-LICES R ; STONE W ; FONTCUBERTA J.** Genetic susceptibility to thrombosis and its relationship to physiological risk factors: the GAIT study. Genetic Analysis of Idiopathic Thrombophilia. *Am J Hum Genet,* 2000, vol. 67 (6), 1452-1459
- **SORIA JM ; MORANGE PE ; VILA J ; SOUTO JC ; MOYANO M ; TRÉGOUËT DA ; MATEO J ; SAUT N ; SALAS E ; ELOSUA R.** Multilocus genetic risk scores for venous thromboembolism risk assessment. *J Am Heart Assoc,* 2014, vol. 3 (5), 1-10
- **MCLNNES G ; DANESHJOU R ; KATSONIS P ; LICHTARGE O ; SRINIVASAN R ; RANA S ; RADI-VOJAC P ; MOONEY SD ; PAGEL KA ; STAM-BOULIAN M.** Predicting venous thromboembolism risk from exomes in the Critical Assessment of Genome Interpretation (CAGI) challenges. *Hum Mutat,* 2019, vol. 40 (9), 1314-1320
- **ZERMATTEN MG ; PANTET O ; GOMEZ F ; SCHNEIDER A ; MEAN M ; MAZZOLAI L ; HUGLI O ; BART P-A ; PAPADIMITRIOU-OLIVGERIS M ; AL-BERIO L.** initiative C-19 IC-C. Utility of D-dimers and intermediate-dose prophylaxis for venous thromboembolism in critically ill patients with COVID-19. *Thromb Res,* 2020, vol. 196, 222-226
- **CHOI JJ ; WEHMEYER GT ; LI HA ; ALSHAK MN ; NAHID M ; RAJAN M ; LIU B ; SCHATOFF EM ; ELAHJJI R ; ABDELGHANY Y.** D-dimer cut-off points and risk of venous thromboembolism in adult hospitalized patients with COVID-19. *Thromb Res,* 2020, vol. 196, 318-321
- **BRUZELIUS M ; BOTTAI M ; SABATER-LLEAL M ; STRAWBRIDGE RJ ; BERGENDAL A ; SILVEIRA A ; SUNDSTRÖM A ; KIELER H ; HAMSTEN A ; ODEBERG J.** Predicting venous thrombosis in women using a combination of genetic markers and clinical risk factors. *J Thromb Haemost,* 2015, vol. 13 (2), 219-227
- **MCDAID A ; LOGETTE E ; BUCHILLIER V ; MURI-SET M ; SUCHON P ; PACHE TD ; TANACKOVIC G ; KUTALIK Z ; MICHAUD J.** Risk prediction of developing venous thrombosis in combined oral contraceptive users. *PLoS One,* 2017, vol. 12 (7), e0182041
- **ROSENDAAL FR.** Causes of Venous Thrombosis. *Deep Vein Thromb Pulm Embolism,* 2009, vol. 14 (1), 1-26
- **HAJIAN-TILAKI K.** Receiver Operating Characteristic (ROC) Curve Analysis for Medical Diagnostic Test Evaluation. *Casp J Intern Med,* 2013, vol. 4 (2), 627-635
- **RUOPP MD ; PERKINS NJ ; WHITCOMB BW ; SCHISTERMAN EF.** Youden Index and optimal cut-point estimated from observations affected by a lower limit of detection. *Biom J,* 2008, vol. 50 (3), 419-430
- **DELONG ER ; DELONG DM ; CLARKE-PEARSON DL.** Comparing the areas under two or more correlated receiver operating characteristic curves: a nonparametric approach. *Biometrics,* 1988, vol. 44 (3), 837-845
- **KAPTEIN FHJ ; STALS MAM ; GROOTENBOERS M ; BRAKEN SJE ; BURGGRAAF JLI ; VAN BUS-SEL BCT ; CANNEGIETER SC ; TEN CATE H ; EN-DEMAN H ; GOMMERS DAMPJ.** Incidence of thrombotic complications and overall survival in hospitalized patients with COVID-19 in the second and first wave. *Thromb Res,* 2021, vol. 199, 143-148
- **SPYROPOULOS AC ; COHEN SL ; GIANOS E ; KOHN N ; GIANNIS D ; CHATTERJEE S ; GOLDIN M ; LESSER M ; COPPA K ; HIRSCH JS.** Group the C-19 C. Validation of the IMPROVE-DD risk assessment model for venous thromboembolism among hospitalized patients with COVID-19. *Res Pract Thromb Haemost,* 2021, vol. 5 (2), 296-300, https://doi.org/10.1002/rth2.12486
- **DUJARDIN RWG ; HILDERINK BN ; HAKSTEEN WE ; MIDDELDORP S ; VLAAR APJ ; THACHIL J ; MULLER MCA ; JUFFERMANS NP.** Biomarkers for the prediction of venous thromboembolism in critically ill COVID-19 patients. *Thromb Res,* 2020, vol. 196, 308-312
- Predicting Venous Thromboembolic Events in Patients with Coronavirus Disease 2019 Requiring Hospitalization: an Observational Retrospective Study by the COVIDIC Initiative in a Swiss University Hospital. **KAMPOURI E ; FILIPPIDIS P ; VIALA B ; MEAN M ; PANTET O ; DESGRANGES F ; TSCHOPP J ; REGINA J ; KARACHALIAS E ; BIANCHI C.** Biomed Res Int. 2020, vol. 2020, 9126148
- **DELANEAU O ; ZAGURY JF ; MARCHINI J.** Improved whole-chromosome phasing for disease and population genetic studies. *Nat Methods,* 2013, vol. 10 (1), 5-6

- **O'DONNELL J ; BOULTON FE ; MANNING RA ; LAFFAN MA.** Amount of H Antigen Expressed on Circulating von Willebrand Factor Is Modified by ABO Blood Group Genotype and Is a Major Determinant of Plasma von Willebrand Factor Antigen Levels. *Arterioscler Thromb Vasc Biol,* 2002, vol. 22 (2), 335-341
- **CORRAL J ; GONZALEZ-CONEJERO R ; SORIA JM ; GONZALEZ-PORRAS JR ; PEREZ-CEBALLOS E ; LECUMBERRI R ; ROLDAN V ; SOUTO JC ; MIÑANOA ; HERNANDEZ-ESPINOSA D.** A nonsense polymorphism in the protein Z-dependent protease inhibitor increases the risk for venous thrombosis. *Blood,* 2006, vol. 108 (1), 177-183
- **POORT SR ; ROSENDAAL FR ; REITSMA PH ; BERTINA RM.** A common genetic variation in the 3'-untranslated region of the prothrombin gene is associated with elevated plasma prothrombin levels and an increase in venous thrombosis. *Blood,* 1996, vol. 88 (10), 3698-3703
- **BERTINA RM ; KOELEMAN BP ; KOSTER T ; ROSENDAAL FR ; DIRVEN RJ ; DE RONDE H ; VAN DER VELDEN PA ; REITSMA PH.** Mutation in blood coagulation factor V associated with resistance to activated protein C. *Nature,* 1994, vol. 369 (6475), 64-67
- **VAN HYLCKAMA VLIEG A ; KOMANASIN N ; ARIËNS RAS ; POORT SR ; GRANT PJ ; BERTINA RM ; ROSENDAAL FR.** Factor XIII Val34Leu polymorphism, factor XIII antigen levels and activity and the risk of deep venous thrombosis. *Br J Haematol,* 2002, vol. 119 (1), 169-175
- **KANAJI T ; OKAMURA T ; OSAKI K ; KUROIWA M ; SHIMODA K ; HAMASAKI N ; NIHO Y.** A common genetic polymorphism (46 C to T substitution) in the 5'-untranslated region of the coagulation factor XII gene is associated with low translation efficiency and decrease in plasma factor XII level. *Blood,* 1998, vol. 91 (6), 2010-2014
- **KLARIN D ; BUSENKELL E ; JUDY R ; LYNCH J ; LEVIN M ; HAESSLER J ; ARAGAM K ; CHAFFIN M ; HAAS M ; LINDSTRÖM S.** Genome-wide association analysis of venous thromboembolism identifies new risk loci and genetic overlap with arterial vascular disease. *Nat Genet,* 2019, vol. 51 (11), 1574-1579
- **LINDSTRÖM S ; WANG L ; SMITH EN ; GORDON W ; VAN HYLCKAMA VLIEG A ; DE ANDRADE M ; BRODY JA ; PATTEE JW ; HAESSLER J ; BRUMPTON BM.** Genomic and transcriptomic association studies identify 16 novel susceptibility loci for venous thromboembolism. *Blood,* 2019, vol. 134 (19), 1645-1657
- **GOUMIDI L ; THIBORD F ; WIGGINS KL ; LI-GAO R ; BROWN MR ; VAN HYLCKAMA VLIEG A ; SOUTO J-C ; SORIA J-M ; IBRAHIM-KOSTA M ; SAUT N.** Association between ABO haplotypes and the risk of venous thrombosis: impact on disease risk estimation. *Blood,* 2021, vol. 137 (17), 2394-2402
- **HOILAND RL ; FERGUSSON NA ; MITRA AR ; GRIESDALE DEG ; DEVINE D V ; STUKAS S ; COOPER J ; THIARA S ; FOSTER D ; CHEN LYC.** The association of ABO blood group with indices of disease severity and multiorgan dysfunction in COVID-19. *Blood Adv,* 2020, vol. 4 (20), 4981-4989
- **DE HAAN HG ; BEZEMER ID ; DOGGEN CJM ; CESSIE S LE ; REITSMA PH ; ARELLANO AR ; TONG CH ; DEVLIN JJ ; BARE LA ; ROSENDAAL FR.** Multiple SNP testing improves risk prediction of first venous thrombosis. *Blood,* 2012, vol. 120 (3), 656-663
- **VOICU S ; KETFI C ; STÉPANIAN A ; CHOUSTERMAN BG ; MOHAMEDI N ; SIGURET V ; MEBAZAA A ; MEGARBANE B ; BONNIN P.** Pathophysiological Processes Underlying the High Prevalence of Deep Vein Thrombosis in Critically Ill COVID-19 Patients. *Front Physiol,* 2020, vol. 11, 608788
- **LOO J ; SPITTLE DA ; NEWNHAM M.** COVID-19, immunothrombosis and venous thromboembolism: biological mechanisms. *Thorax,* 2021, vol. 76 (4), 412-420
- **ABDOL RAZAK NB ; JONES G ; BHANDARI M ; BERNDT MC ; METHAROM P.** Cancer-Associated Thrombosis: An Overview of Mechanisms, Risk Factors, and Treatment. *Cancers (Basel),* 2018, vol. 10 (10), 380
- **PABINGER I ; AY C ; DUNKLER D ; THALER J ; REITTER E-M ; MAROSI C ; ZIELINSKI C ; MANNHALTER C.** Factor V Leiden mutation increases the risk for venous thromboembolism in cancer patients - results from the Vienna Cancer And Thrombosis Study (CATS). *J Thromb Haemost,* 2015, vol. 13 (1), 17-22
- **MUÑOZ MARTIN AJ ; ORTEGA I ; FONT C ; PACHÓN V ; CASTELLÓN V ; MARTÍNEZ-MARÍN V ; SALGADO M ; MARTINEZ E ; CALZAS J ; RUPÉREZ A.** Multivariable clinical-genetic risk model for predicting venous thromboembolic events in patients with cancer. *Br J Cancer,* 2018, vol. 118 (8), 1056-1061